(19) Europäisches Patentamt / European Patent Office / Office européen des brevets

(11) **EP 3 431 490 B1**

(12) **EUROPEAN PATENT SPECIFICATION**

(45) Date of publication and mention
of the grant of the patent:
**11.03.2020 Bulletin 2020/11**

(51) Int Cl.:
*C07K 5/103* (2006.01)     *C07K 5/11* (2006.01)
*G01N 33/68* (2006.01)     *G01N 33/52* (2006.01)

(21) Application number: **18183815.2**

(22) Date of filing: **16.07.2018**

(54) **CHEMICAL COMPOUNDS FOR USE AS DIAGNOSTIC MARKERS OF INFLAMMATION AND NEOPLAMS, THE METHOD FOR SYNTHESIS OF THE CHEMICAL COMPOUNDS, DIAGNOSTIC KIT FOR USE IN DIAGNOSIS OF INFLAMMATORY PROCESSES AND EPITHELIAL NEOPLASMS AND IN VITRO DIAGNOSTIC METHOD OF INFLAMMATORY PROCESSES AND EPITHELIAL NEOPLASMS**

CHEMISCHE VERBINDUNGEN ZUR VERWENDUNG ALS DIAGNOSTISCHE MARKER VON ENTZÜNDUNGEN UND NEOPLASMEN, VERFAHREN ZUR SYNTHESE DER CHEMISCHEN VERBINDUNGEN, DIAGNOSTISCHES KIT ZUR VERWENDUNG BEI DER DIAGNOSE VON ENTZÜNDLICHEN PROZESSEN UND EPITHELIALEN NEOPLASMEN SOWIE DIAGNOSTISCHES IN-VITRO-VERFAHREN VON ENTZÜNDLICHEN PROZESSEN UND EPITHELIALEN NEOPLASMEN

COMPOSÉS CHIMIQUES UTILISÉS COMME MARQUEURS DIAGNOSTIQUES D'INFLAMMATIONS ET DE TUMEURS, PROCÉDÉ DE SYNTHÈSE DE COMPOSÉS CHIMIQUES, KIT DE DIAGNOSTIC À UTILISER DANS LE DIAGNOSTIC DE PROCESSUS INFLAMMATOIRES ET DE NÉOPLASIES ÉPITHÉLIALES ET PROCÉDÉ DE DIAGNOSTIC IN VITRO DE PROCESSUS INFLAMMATOIRES ET DES NÉOPLASIES ÉPITHÉLIALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2017 PL 42223317**
**14.07.2018 PL 42633218**

(43) Date of publication of application:
**23.01.2019 Bulletin 2019/04**

(73) Proprietor: **Uniwersytet Gdanski**
**80-309 Gdansk (PL)**

(72) Inventors:
• **Lesner, Adam**
**80-288 Gdansk (PL)**
• **Gruba, Natalia**
**84-241 Goscicino (PL)**

(74) Representative: **Pawlowska, Justyna**
**Kancelaria Patentowa Justyna Pawlowska**
**Wzgorze Bernardowo 263B/6**
**81-583 Gdynia (PL)**

(56) References cited:
**WO-A1-2009/154510     WO-A2-03/064620**
**PL-B1- 225 341**

• **NATALIA GRUBA ET AL: "Bladder cancer detection using a peptide substrate of the 20S proteasome", FEBS JOURNAL, vol. 283, no. 15, 1 August 2016 (2016-08-01), pages 2929-2948, XP055529798, GB ISSN: 1742-464X, DOI: 10.1111/febs.13786**

**Description**

The field of the invention is diagnosis *in vitro* of inflammatory processes, the mechanism initiates *in vivo* as a result of immune responses of immune system blood cells and diagnostics of neoplasms *in vitro,* especially epithelial neoplasms in particular tumors. The invention relates to markers for use in *in vitro* diagnosis of inflammatory processes and diagnostic markers for use in in vitro diagnosis of epithelial neoplasms at a very early stage. The invention relates to compounds for diagnostic use - for use in diagnosis of inflammatory conditions and in the early diagnosis of epithelial neoplasms, wherein these compounds are used as a diagnostic agent - diagnostic marker - to detect the pathophysiological process in the biological material of mammals, especially human blood and urine *in vitro.* The invention relates to a diagnostic kit containing at least two new compounds - a diagnostic reagent for use in detecting immune changes, including predicting the development of epithelial cell neoplasms at a very early stage of the disease, even before the process of neoplasm processes - in diagnosis of inflammatory process, or for use in diagnosis of initiated neoplasms process with simultaneous inflammatory reaction, and a marker for use in the detection of additional inflammatory processes regardless of presence of epithelial neoplasms, e.g. bacterial or viral infection that is additionally present. The invention also relates to a method for the preparation of new compounds based on a chain of amino acids while the compounds are used for diagnosis of inflammatory processes and / or neoplasms especially epithelial neoplasms.

There are known diagnostic compounds and markers for detecting inflammation as well as neoplasms formation in particular tumorigenesis. Peptides for detecting bladder cancer from patent publications such as WO 2011/038142 or WO 2014/042209 or from journal articles such as Gruba et al., FEBS Journal, vol. 283, pages 2929-2948 (2016) are known.

The disclosed agents by marking it with detecting markers, allow detection of the disease state.

Due to the fact that late inflammation diagnosis that directly leading to the development of neoplasm - cancer is associated with a reduction in the chance of treatment success and even cure or is associated with therapeutic difficulties, new diagnostic compounds are still sought for sensitively and specifically to detect disease processes, especially inflammation correlated with neoplasms as soon as in the earliest stages of the pathophysiological process.

The object of the invention is to provide a sensitive and specific diagnostic markers for use in diagnosis of a neoplasm in particular tumor, epithelial cancer and including bladder cancer, urinary tract cancer, urethral cancer or other cancers accompanied by an increase in proteolytic enzymes, and which can be observed in the disease process in vitro in the biological material, especially the urine of the patient, at a very early stage of pathogenesis, i.e. even at the inflammatory reaction stage that are direct cause of neoplasm formation. Unexpectedly, it has been found that the new amino acid-containing compounds developed during the invention study are useful as a diagnostic markers that only in the presence of proteolytic enzymes that are the result of an inflammatory reaction and / or neoplasms e.g. epithelial neoplasms, tumor, and which enzymes are released and present in the biological material, exhibit chromogenic properties and/or fluorogenic measured in the 300-500 nm waveband, especially 380-430. The color properties are observed during the enzymatic hydrolysis of these compounds in the biological material in vitro, especially the urine and blood of the patient in whom the epithelial neoplasms is present or is during formation, including the inflammatory process or whether the inflammatory response itself or an inflammatory response additionally accompanied by neoplastic disease has developed. In the case of neoplasm, this process is associated with the effects of neoplasms - cancer, i.e. an increase in the activity of proteolytic enzymes of the tumor and surrounding tissues resulting from many processes, including increased angiogenesis, tissue remodeling, necrosis of healthy tissues due to tumor infiltration and tumor cell apoptosis. Part of the compounds according to the invention makes it possible to detect even the already inflammatory process, also by hydrolysing process, including the release of enzymes from the inflammatory focus during the only immunological process, which involves triggering a color reaction detected by measuring the absorbance in the 300-500 nm wavelength range, especially 380-430, especially 410 nm.

During the research conducted in the Laboratory of Biochemical Analytics and Nanodiagnostics of the University of Gdansk in Poland, the identification of compounds that are susceptible to increased proteolytic activity in biological material, in particular human urine, used as diagnostic markers of epithelial neoplasms in particular tumors, urinary epithelial carcinoma and diagnostic markers of inflammatory processes, has been carried out. In the course of the research, an experimental study was carried out on human biological material, including urine of patients with confirmed inflammatory reaction and / or epithelial neoplasms in particular tumors at various stages of development, as well as urine of people with excluded inflammation - control group.

As a result of these studies, compounds with the sequence ABZ-Leu-Glu-Pro-Val-ANB-$NH_2$ were developed, where ABZ is 2-aminobenzoic acid and ANB-NH2 is 5-amino-2-benzoic acid amide, ANB is the 5-amino-2-benzoic acid, and ABZ-Leu-Glu-Pro-Val-pNA, where pNA is para-nitroaniline. These compounds are for use as diagnostic markers for the detection of the immune process - - inflammatory response as well as the diagnostic markers for neoplasms in particular epithelial neoplasms detection. The study also developed a compound of the formula ABZ-Met-Lys-Val-Trp-pNA, where ABZ is 2-aminobenzoic acid and pNA is para-nitroaniline, and ABZ-Met-Lys-Val-Trp-ANB-$NH_2$, where ABZ is 2-aminobenzoic acid, and ANB - 5-amino-2-benzoic acid. The compounds are for use as markers for detecting neoplasms in particular epithelial neoplasms, cancer. The inflammatory process detected by the compounds mentioned above as first may also be correlated at the time of the appearance of the neoplasm around which the inflammation occurs. Detection of inflammation may also be useful in the case of infection with bacteria or viruses in the presence or absence of neoplasms in particular epithelial neoplasms.

Both processes diagnosed by the proposed compounds are accompanied by / results of enzymatic hydrolysis of these markers - as a result of the release of enzymes during immunological processes - inflammatory process, taking place in vivo and/or proteolytic enzymes released as a result of the development of neoplasms in particular epithelial neoplasms including cancer. This process is observed by monitoring the released $ANB-NH_2$ molecule (5-amino-2-benzoic acid amide) or pNA (p-nitroaniline) at a wavelength of 300-400, preferably 380-430 nm, respectively, that is, hydrolysis occurs at the 5th position of the compound - the 5th amino acid in the sequence.

The present invention provides a compound defined by the general formula: $ABZ^1\text{-}Leu^2\text{-}Glu^3\text{-}Pro^4\text{-}Val^5\text{-}X^6$, wherein: ABZ denotes 2-aminobenzoic acid, X is $ANB-NH_2$ or pNA, where $ANB-NH_2$ denotes 5-amino-2-benzoic acid amide, ANB - 5-amino-2-benzoic acid and pNA denotes 4-nitroaniline.

The present invention also provides a compound of the formula: $ABZ^1\text{-}Met^2\text{-}Lys^3\text{-}Val^4\text{-}Trp^5\text{-}X^6$, wherein: ABZ is 2-aminobenzoic acid, X is $ANB-NH_2$ or pNA, where $ANB-NH_2$ is 5-amino-2-benzoic acid amide, ANB - 5-amino-2-benzoic acid and pNA means 4-nitroaniline.

The present invention also provides a process for the preparation of $ABZ^1\text{-}Leu^2\text{-}Glu^3\text{-}Pro^4\text{-}Val^5\text{-}ANB\text{-}NH_2^6$, where: ABZ is 2-aminobenzoic acid, $ANB-NH_2$ is 5-amino-2-benzoic acid amide, and this compound is obtained in the synthesis on a solid support and the method is carried out in the following steps:

a) preparation of a resin enabling the conversion of an acid into an amide,
b) deprotection - removal of the protective group,
c) deposition of 5-amino-2-nitrobenzoic acid ANB,
d) repeating steps from b) to c) until a compound is obtained, wherein the synthesis is carried out from residues 6 to 1, followed by cleavage of the obtained peptide from the resin.

The invention also relates to a process for the preparation of the compound $ABZ^1\text{-}Leu^2\text{-}Glu^3\text{-}Pro^4\text{-}Val^5\text{-}pNA^6$, where: ABZ is 2-aminobenzoic acid, pNA is 4-nitroaniline, and the compound is obtained in the synthesis partly on solid support and partly in buffer or only on a solid support, and the method is carried out in the following steps:

a) preparation of 2-chloro-chlorotrityl resin,
b) deprotection of the protective group,
c) embedding of Pro4 on the resin,
d) repeating steps from b) to c) until obtaining ABZ-Leu-Glu-Pro;
e) cleavage of the obtained peptide from the resin
f) obtaining the amino acid paranitroanilide (Val-pNA)
g) coupling of paranitroanilide with a protected peptide (ABZ-Leu-Glu-Pro + Val-pNA,
h) removing the side groups of amino acids.

The invention also relates to a process for the preparation of $ABZ^1\text{-}Met^2\text{-}Lys^3\text{-}Val^4\text{-}Trp^5\text{-}ANB\text{-}NH_2^6$, where: ABZ is 2-aminobenzoic acid, $ANB-NH_2$ is 5-amino-2-benzoic acid amide and the compound is obtained in solid phase synthesis and the method is carried out in the following steps:

a) preparation of a resin enabling the conversion of an acid into an amide,
b) deprotection of the protective group,
c) deposition of 5-amino-2-nitrobenzoic acid ANB,
d) repeating steps from b) to c) until a compound is obtained, wherein the synthesis is carried out from residues 6 to 1, followed by cleavage of the obtained peptide from the resin.

The invention also relates to a process for the preparation of the compound $ABZ^1\text{-}Met^2\text{-}Lys^3\text{-}Val^4\text{-}Trp^5\text{-}pNA^6$, where: ABZ is 2-aminobenzoic acid and pNA is 4-nitroaniline, and the compound is obtained in solid phase synthesis and partly in buffer or only on a solid support and the method is carried out in the following steps:

a) preparation of 2-chloro-chlorotrityl resin,
b) deprotection of the protective group,
c) embedding $Val^4$ on the resin,
d) repeating steps from b) to c) until obtaining $ABZ^1\text{-}Met^2\text{-}Lys^3\text{-}Val^4$;
e) cleavage of the obtained peptide from the resin

f) obtaining the amino acid paranitroanilide (Trp-pNA)

g) coupling of paranitroanilide with a protected peptide (ABZ-Leu-Glu-Pro + Trp-pNA,

h) removing the side groups of amino acids.

The subject of the invention is a diagnostic marker of the general formula ABZ$^1$-Leu$^2$-Glu$^3$-Pro$^4$-Val$^5$-X$^6$, where: ABZ is 2-aminobenzoic acid, X is ANB-NH$_2$ or pNA, where ANB-NH$_2$ is 5-amino-2-benzoic acid amide, pNA is 4-nitroaniline, for use in the *in vitro* diagnosis of inflammation and/or neoplasms in particular cancer, especially epithelial neoplasms.

The subject of the invention is a diagnostic marker of the general formula ABZ$^1$-Met$^2$-Lys$^3$-Val$^4$-Trp$^5$-X$^6$, where: ABZ is 2-aminobenzoic acid and X is ANB-NH$_2$ or pNA, wherein ANB-NH$_2$ is 5-amino-2-benzoic acid amide, pNA is 4-nitroaniline, for use in the *in vitro* diagnosis of neoplasms in particular cancer, especially epithelial neoplasms.

The subject of the invention is a diagnostic kit for use in the *in vitro* early diagnosis of neoplasm, especially epithelial neoplasms and/or inflammatory disease, comprising at least one compound of general formula ABZ$^1$-Leu$^2$-Glu$^3$-Pro$^4$-Val$^5$-X$^6$ and at least one compound of formula ABZ$^1$-Met$^2$-Lys$^3$-Val$^4$-Trp$^5$-X$^6$, where: ABZ is 2-aminobenzoic acid, X is ANB-NH$_2$ or pNA, where ANB-NH$_2$ is 5-amino-2-benzoic acid amide, pNA is 4-nitroaniline.

The subject of the invention is a method for *in vitro* diagnosing epithelial cancer and/or an inflammatory process in a mammalian biological material, characterized in that as a diagnostic marker, at least one compound of the general formula ABZ$^1$-Leu$^2$-Glu$^3$-Pro$^4$-Val$^5$-X$^6$ is for use a marker of the immune response and additionally for diagnosis of neoplasms, where: ABZ is 2-aminobenzoic acid, X is ANB-NH$_2$ or pNA, where ANB-NH$_2$ is 5-amino-2-benzoic acid amide, pNA is 4-nitroaniline. This compound is added and incubated in the biological material of the mammal, especially urine or human blood, then absorbance is measured at a wavelength in the range of 300 to 500 nm. Demonstration of color and increase in absorbance indicates a positive result. The color is the result of the enzymatic hydrolysis of the compound especially in the position 5 - after the fifth amino acid in turn, under the influence of proteolytic enzymes present in the biological material in vitro as a result of an immune response and/or neoplasms in vivo. The absorbance reading time is determined depending on the amount of compound added. The reading can take place after about 60 minutes.

Preferably, the absorbance is measured at 380-430 nm.

The subject of the invention is a method for *in vitro* diagnosing of epithelial neoplasm in biological material of a mammal, characterized according to the invention in that at least one compound of the general formula ABZ$^1$-Met$^2$-Lys$^3$-Val$^4$-Trp$^5$-X$^6$ is for use as a marker of neoplasm - the diagnostic marker; where: ABZ is 2-aminobenzoic acid, X is ANB-NH$_2$ or pNA, where ANB-NH$_2$ is 5-amino-2-benzoic acid amide, pNA is 4-nitroaniline. The compound is added to the biological material, then absorbance is measured at a wavelength in the range of 300 to 500 nm. Demonstration of color and increase in absorbance indicates a positive result. The color is the result of the enzymatic hydrolysis of the compound, especially at the position 5 - after the fifth amino acid in turn, under the influence of proteolytic enzymes present in the biological material *in vitro* as a result of the *in vivo* tumor-related process. The absorbance reading time is determined depending on the amount of compound added. The reading can take place after about 60 minutes.

Preferably, the absorbance is measured at 380-430 nm.

The method for *in vitro* diagnosing and differentiating epithelial neoplasms and the inflammatory process in a mammalian biological material according to the invention is characterized in that at least one compound of the general formula ABZ$^1$-Leu$^2$-Glu$^3$-Pro$^4$-Val$^5$-X$^6$ is for use as a diagnostic marker - the marker for use in diagnosing of the immune response and in addition a neoplasm, and at least one compound of the general formula ABZ$^1$-Met$^2$-Lys$^3$-Val$^4$-Trp$^5$-X$^6$ I for use as a marker for neoplasm diagnosing; wherein ABZ is 2-aminobenzoic acid, X is ANB-NH$_2$ or pNA, where ANB-NH$_2$ is 5-amino-2-benzoic acid amide, pNA is 4-nitroaniline. The compounds are added to the biological material, each into a separate sample, then absorbance is measured at a wavelength in the range of 300 to 500 nm. The increase in absorbance indicates a positive result. The color is the result of the enzymatic hydrolysis of the compound especially in the position 5 - after the fifth amino acid in turn, under the influence of proteolytic enzymes present in the biological material in vitro as a result of an immune response and/or tumor in vivo. The absorbance reading time is determined depending on the amount of compound added. The reading can take place after about 60 minutes.

Preferably, the absorbance is measured at 380-430 nm.

A method for the preparation of ABZ$^1$-Leu$^2$-Glu$^3$-Pro$^4$-Val$^5$-ANB-NH$_2$$^6$, wherein: ABZ is 2-aminobenzoic acid, ANB-NH$_2$ is 5-amino-2-benzoic acid amide, according to the embodiment, the method is carried out in the following stages: swelling of the amide resin and:

a) deprotection - removing the Fmoc shield,

b) washing the resin,

c) deposition of 5-amino-2-nitrobenzoic acid,

d) washing the resin,

e) repeating steps from b) to d) until the compound/peptide is obtained; the synthesis is from C from the N-terminus (from the rest 6 to 1),
f) cleavage of the obtained peptide from the resin, removal of the covers.

A method for the preparation of the compound $ABZ^1$-$Leu^2$-$Glu^3$-$Pro^4$-$Val^5$-$pNA^6$, wherein: ABZ is 2-aminobenzoic acid, pNA means para-nitroaniline, according to embodiment is carried out in the following steps:

a) swelling process of 2-chloro-chlorotritric resin
b) deprotection - removing the Fmoc shield,
c) washing the resin,
d) embedding of $Pro^4$ on the resin,
e) washing the resin,
f) repeating steps from b) to e) until obtaining ABZ-Leu-Glu-Pro,
g) cleavage of the obtained peptide from the resin,
h) obtaining the amino acid paranitroanilide (Val-pNA),
i) coupling of paranitroanilide with a protected peptide (ABZ-Leu-Glu-Pro + Val-pNA,
j) removing the side groups of amino acids (Glu(tBu).

In a similar manner as described above, in the embodiment, a compound of the formula $ABZ^1$-$Met^2$-$Lys^3$-$Val^4$-$Trp^5$-$pNA^6$ is obtained, wherein: ABZ is 2-aminobenzoic acid and pNA is 4-nitroaniline using other amino acids. The compound is obtained in the synthesis on a solid support and the method is carried out in the following steps:

a) preparation of 2-chloro-chlorotrityl resin,
b) deprotection of the protective group, e.g. Fmoc,
c) embedding $Val^4$ on the resin,
d) repeating steps from b) to c) until obtaining $ABZ^1$-$Met^2$-$Lys^3$-$Val^4$;
e) cleavage of the obtained peptide from the resin
f) obtaining the amino acid paranitroanilide (Trp-pNA)
g) coupling of paranitroanilide with a protected peptide (ABZ-Leu-Glu-Pro + Trp-pNA,
h) removing the side groups of amino acids (Glu(tBu).

The method of preparing the compound $ABZ^1$-$Met^2$-$Lys^3$-$Val^4$-$Trp^5$-$ANB$-$NH_2$, in an embodiment, is carried out in the following steps:

a) the swelling process of the amide resin;
b) deprotection of the Fmoc shield;
c) washing the resin;
d) performing a chloranil test;
e) deposition of 5-amino-2-nitrobenzoic acid;
f) washing the resin;
g) performing a chloranil test;
h) repeating steps from b) to g) until a compound is obtained;
i) cleavage of the peptide from the resin while removing the shields.

In the four methods described, deprotection of the Fmoc sheath is carried out especially in the piperidine solution, especially 20% in NMP, the resin washing is carried out especially in DMF, piperidine and/or DMF and DCM, the removal of the peptide from the resin while removing the shields is carried out especially by mixtures of TFA: phenol: water : TIPS, especially in the ratio of 88 : 5 : 5 : 2, v / v / v / v. After detachment of the peptide, filtration, centrifugation, dissolution of the precipitate in distilled water by means of ultrasounds, followed by lyophilization are carried out. Then, analysis, confirmation of the obtained compound and purification are carried out, e.g. by means of HPLC, MS, NMR and in particular mass spectrometry to confirm identity.

The invention enables the detection of epithelial neoplasm at an early stage of development - in biological material *in vitro* - especially urine or blood. The invention additionally allows for the diagnosis of an immune response independently or in addition to neoplams. The compounds of the invention find use as markers already for the detection of the immune process - the immune response as well as the diagnostic markers of epithelial neoplasm, especially tumors. The inflammatory process detected by the compounds of the invention may also be associated with the development of neoplams around which inflammation occurs. Detection of inflammation may also be useful in the case of infection with bacteria or viruses in the presence or absence of epithelial neoplasm in particular cancer. Research results indicate that these compounds are very sensitive and precise markers of epithelial neoplasm, especially tumors, for the diagnosis of neoplasm development, even at a very early stage. The diagnostic kit according to the invention makes it possible to simultaneously conduct diagnostics of inflammatory processes - the immune response as well as the diagnosis of epithelial neoplasm especially tumor.

Part of the terms used in the description and claims have the following meanings:

Epithelial neoplasms in particular tumor - means the one whose cells originate from the epithelium of the external and internal secretion glands and from the glands of these glands, e.g. epithelial tumor of the urinary tract.
Chromogenic properties - means the formation of a colored product
Fluorogenic properties - means the formation of a fluorescent product
NMP - $N$-methylpyrrolidone
DMF - dimethylformamide
DCM - methylene chloride
pNA-4-nitroaniline, para-nitroaniline
ABZ - 2-aminobeznoesic acid,
ANB-NH$_2$ - 5-amino-2-benzoic acid amide,
Boc - tert-butyloxycarbonyl group
Fmoc - 9-fluorenylmethoxycarbonyl group
TFA - trifluoroacetic acid.

The invention is shown in more detail in the embodiments and in the drawing, in which:

Fig. 1 - shows the rate of hydrolysis of the ABZ-Leu-Glu-Pro-Val-ANB-NH2 substrate in urine samples diagnosed with neoplasm. Arabic numerals are the number of the randomly selected urine sample.

Fig. 2 - shows the rate of hydrolysis of the ABZ-Leu-Glu-Pro-Val-pNA substrate in urine samples diagnosed with neoplasm. Arabic numerals are the number of the randomly selected urine sample.

Fig. 3 - shows the hydrolysis rates of the ABZ-Leu-Glu-Pro-Val-ANB-NH$_2$ substrate depending on the presence of an inhibitor of human neutrophilic elstasis, which enzyme is a cross-fertilization marker in a human urine sample with confirmed inflammation and/or neoplasm of the epithelium of the urinary tract.

Fig. 4 - shows the dependence of the degree of hydrolysis of the ABZ-Leu-Glu-Pro-Val-ANB NH$_2$ substrate from the pH environment

Fig. 5 - shows the hydrolysis efficiency of the ABZ-Leu-Glu-Pro-Val-ANB-NH$_2$ substrate depending on the degree of compaction of human urine with the confirmed urinary epithelial carcinoma.

Fig. 6 - shows the hydrolysis rate of the ABZ-Met-ys-Val-Trp-ANB-NH$_2$ substrate in urine samples diagnosed with cancer. Arabic numerals are the number of the randomly selected urine sample.

Fig. 7 - shows the hydrolysis rates of the ABZ-Met-Lys-Val-Trp-ANB-NH$_2$ substrate depending on the presence of the inhibitor in a human urine sample with a confirmed epithelial tumor of the urinary tract.

Fig. 8 - shows the dependence of the degree of hydrolysis of the ABZ-Met-Lys-Val-Trp-ANB-NH$_2$ substrate from the pH environment

Fig. 9 - shows the hydrolysis efficiency of the ABZ-Met-Lys-Val-Trp-ANB-NH$_2$ substrate depending on the degree of compaction of human urine with a confirmed epithelial neoplasms of the urinary tract.

The invention is illustrated by the following non-limiting examples.

Example 1

Synthesis of a new compound ABZ[1]-Leu[2]-Glu[3]-Pro[4]-Val[5]-ANB-NH$_2$[6]

1. Obtaining a chromogenic peptide

a) The first stage of the synthesis was to obtain a chromogenic peptide that was obtained by solid phase synthesis - on a solid support - using Fmoc/tBu chemistry, i.e. using covers.

A compound with the sequence ABZ[1]-Leu[2]-Glu[3]-Pro[4]-Val[5]-ANB-NH$_2$[6], where ABZ is 2-aminobenzoic acid and ANB-NH$_2$ is 5-amino-2-benzoic acid amide, and ANB is 5-amino-2-benzoic acid, obtained by solid phase chemical synthesis using the following amino acid derivatives.
Boc-ABZ, Fmoc-Leu, Fmoc-Glu(Trt), Fmoc-Pro, Fmoc-Val, ANB
The synthesis of a compound or diagnostic marker for the detection of epithelial neoplasms whose diagnosis is associated with the hydrolysis of this compound under the influence of proteolytic enzymes was carried out on a solid support enabling the conversion of 5-amino-2-benzoic acid into the ANB-NH$_2$ amide:
- e.g. an amide resin, e.g. TentaGel S RAM from RAPP Polymere (Germany) e.g. with a deposition of 0.23 mmol/g.
It is possible to use another commercially available amide resin, including Rink Amide (Germany).
The compound was synthesized in a manual manner using a laboratory shaker. For most of the stages, the reactors were a syringe ended with sinter for synthesis in a solid phase with a capacity of 25 ml.
All final compounds obtained contained in their sequence at the 1-position, i.e. at the N-terminus, 2-aminobenzoic acid - ABZ and at the 6-position: the 5-amino-2-nitrobenzoic acid molecule ANB at the C-terminus. ABZ acts as a fluorescence donor, whereas ANB - 5-amino-2-benzoic acid acts as a quencher of fluorescence and simultaneously a chromophore. The peptides in their sequence contained at least and preferably one reactive site located between the amino acid residues Val-ANB-NH$_2$: at the 5-position of the compound. The synthesis involving the attachment of amino acid derivatives is carried out from residue 6 to 1, i.e. from the end of C to N.

b) Deposition of ANB on TentaGel S RAM resin:

The peptide synthesis was performed on a Rapp Polymere resin TentaGel S RAM with a deposition of 0.23 mmol/g. In the first stage, a resin was prepared, including the loosening of the resin through a series of washes. Subsequent removal of the amine Fmoc protecting group from the carrier was carried out with a 20% piperidine solution in NMP. Then a cycle of solvent washes was carried out. In order to confirm the presence of free amino groups, a chloranil test was carried out.

Washing cycle with solvents:

DMF 1 x 10 minutes
IsOH 1 x 10 minutes
DCM 1 x 10 minutes

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

c) Chloranil test:

The chloranil test consisted of transferring, by means of a spatula, several resin beads from the reactor - a syringe into a glass ampoule, to which was then added 100μl of a saturated solution of p-chloranil in toluene and 50μl of fresh acetaldehyde. After 10 minutes, the color control of the grains was carried out.

At this stage, after the test, green color of the grains was obtained, which indicated the presence of free amine groups. After confirming the removal of the 9-fluorenylmethoxycarbonyl guards from the resin, the next step could be taken to attach the ANB derivative (5-amino-2-nitrobenose acid)

d) Deposition of 5-amino-2-nitrobenzoic acid on a solid support

The first step in the synthesis of the peptide library - a mixture of peptides was the deposition of ANB per 1g of resin. Prior to attaching the chromophore, the resin used for the reaction was washed with the following solvents: DMF, DCM and again DMF, after which the Fmoc-shield was removed from the carrier functional group. One Fmoc shield removal cycle included the following steps:
Removal of the Fmoc cover:

    20% piperidine in NMP 1 x 3 minutes
    20% piperidine in NMP 1 x 8 minutes

e) Washing

DMF 3 × 2 minutes IsOH 3 × 2 minutes DCM 3 × 2 minutes

f) Chloranil test for the presence of free amino groups.

The amine free resin was washed with 5% *N*-methylmorpholine (NMM) in DMF followed by DMF. The Fmoc shield removal procedure and the wash cycle were performed in a Merrifield dish. ANB was dissolved in DMF in a separate flask and TBTU, DMAP and finally diisopropylethylamine (DIPEA) were added in the following excess to the polymer deposition: ANB/TBTU/DMAP/DIPEA, 3: 3: 2: 6. The mixture thus prepared was added to the resin and stirred for 3 hours. The resin was filtered under reduced pressure, washed with DMF, DCM and isopropanol, and the acylation procedure was repeated two times. To carry out subsequent ANB-attachment reactions, *O*-(7-azabenzotriazol-1-yl)-*N,N,N',N'*-tetramethyluronium hexafluorophosphate (HATU) was used for the resin, and then *O*-(benzotriazol-1-yl)-*N,N,N'N*-tetramethyluronium hexafluorophosphate (HBTU). In the last step, the resin was washed successively with DMF, DCM and isopropanol and dried in the air.

g) Attachment of the C-terminal amino acid residue to the ANB

The corresponding amino acid derivative (9-fold molar excess relative to the resin deposition) was dissolved in pyridine and transferred to a flask containing ANB embedded resin. The whole was cooled to -15°C (ice bath: 1 part $NH_4Cl$ weight, 1 part $NaNO_3$ weight, 1 part ice weight). After reaching the desired temperature, $POCl_3$ (in a 1:1 ratio to the amount of amino acid derivative used) was added and the mixture was stirred on a magnetic stirrer: 20 minutes at -15°C, 30 minutes at room temperature, and 6 hours at 40°C (oil bath). After completion of the reaction, the resin was filtered under reduced pressure, washed with DMF and MeOH and allowed to dry.
In the next step, the rest was attached to the P2 position (proline)
All attachment of amino acid residues was preceded by washing the resin with DMF for 5 minutes. Diisopropylcarbodiimide was used as coupling agent in the subsequent attachments. The procedure was repeated twice.
After each acylation, a resin wash cycle was started, followed by a chloranil test to monitor the attachment of the amino acid derivative to the free amino groups of the resin.
Washing cycle with solvents:

    DMF 3 x 2 minutes
    IsOH 3 x 2 minutes
    DCM 3 x 2 minutes

Chloranil test:

As a result of the tests carried out after the first two couplings, the grain color was first green and then gray, so it was necessary to perform another acylation, as a result of which the resin grains tested with the chloranil test were colorless. This showed the attachment of ANB to the TentaGel S RAM resin, which allowed the transition to the next stage of peptide synthesis.

h) Attachment of consecutive protected amino acid residues:

The resin together with the attached ANB residue in the reactor was washed with DMF and then the Fmoc shield from the amine group was deprotected to attach the protected amino acid derivative of glycine.

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

Chloranil test:

As a result of the chloranil test, a positive result was obtained, as evidenced by the green color of the resin grains. This allowed the start of the next stage - attachment of the Fmoc-Glu (Trt) -OH amino acid residue.
Attachment of an amino acid derivative:
Performed couplings were preceded by washing the resin in DMF. When coupling the protected glutamic acid residue, the composition of the coupling mixture remained unchanged.
At the end of each acylation, a cycle of solvent washes was performed according to the given procedure, followed by a chloranil test for the presence of free amino groups in the solution.
Washing cycle with solvents:

DMF 3 x 2 minutes
IsOH 3 x 2 minutes
DCM 3 x 2 minutes

Chloranil test:

The resin grains during the test after the second acylation were colorless, which allowed for the transition to the next stage of the synthesis, which was the introduction of another protected amino acid derivative - leucine and 2-aminobenzoic acid molecule. The coupling processes were carried out according to the procedure discussed earlier.
Tests carried out after connecting the above-mentioned residues showed positive results, the resin grains were colorless.

Removal of the peptide from the carrier

Upon completion of the synthesis, the ABZ-Leu-Glu-Pro-Val-ANB-$NH_2$ peptide amide was removed from the support with the simultaneous removal of the side shields by means of a mixture of: TFA : phenol : water : TIPS (88 : 5 : 5 : 2, v / v / v / v) in a round bottom flask on a magnetic stirrer.
After 3 hours, the contents of the flask were filtered off under vacuum on Schott sintered frit and washed with diethyl ether. The resulting precipitate was centrifuged on a SIGMA 2K30 centrifuge (Laboratory Centrifuges) for 20 minutes. The precipitate obtained after centrifugation is dissolved in water using ultrasound and then lyophilized.

## Identity / characteristics of a new compound – HPLC analysis, MS

HPLC conditions: RP Bio Wide Pore Supelco C8 column 250 mm 4 mm, phase system A 0.1%
TFA in water B: 80% acetonitrile in A), flow 1 ml/min, UV detection at 226 nm. The connection was confirmed.

Example 2

Preparation of a compound of formula ABZ[1]-Leu[2]-Glu[3]-Pro[4]-Val[5]-pNA[6].

The process is carried out in a similar manner as described in example 1 except that the corresponding amino acid derivatives and additional substituents are used and the process is carried out partially in solution on a solid support.

Preparation of p-nitroanilide Val

a. The first step in the synthesis was to obtain a protected peptide that was obtained by solid phase synthesis using Fmoc/tBu chemistry.

Compound ABZ[1]-Leu[2]-Glu (OtBu)[3]-Pro[4]-OH, where ABZ - is 2-aminobenzoic acid, obtained by solid phase chemical synthesis using the following amino acid derivatives: Boc-ABZ, Fmoc-Leu, Fmoc-Glu (OtBu), Fmoc-Pro.

The synthesis of the compound was carried out on a solid support:

-  2-chloro-chlorotinyl resin, e.g. from Iris BIOTECH GMBH (Germany) with deposition of 1.6 mmol Cl/g groups

The synthesis of the compound was carried out manually using a laboratory shaker. A syringe ended with sinter for solid-phase synthesis with a capacity of 25 ml was the reactor by all stages.

Peptide synthesis was performed on a carrier: 2-chloro-chlorotinyl resin, e.g. Iris BIOTECH GMBH (Germany) with deposition of 1.6 mmol Cl/g groups. In the first stage, the resin was loosened through a series of washes. Subsequent removal of the amine Fmoc protecting group from the carrier was carried out with a 20% piperidine solution in NMP. Then a cycle of solvent washes was carried out. In order to confirm the presence of free amino groups, a chloranil test was carried out.

Washing cycle with solvents:

DMF 1 x 10 minutes
IsOH 1 x 10 minutes
DCM 1 x 10 minutes

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

b) Chloranil test:

The chloranil test consisted of transferring, by means of a spatula, several resin beads from the reactor - a syringe into a glass ampoule, to which was then added 100 $\mu$l of a saturated solution of p-chloranil in toluene and 50 $\mu$l of fresh acetaldehyde. After 10 minutes, the color control of the grains was carried out.

At this stage, after the test, green color of the grains was obtained, which indicated the presence of free amine groups. After confirming the removal of the 9-fluorenylmethoxycarbonyl protections from the resin, it was possible to proceed to the next step - attaching the Fmoc-Pro derivative.

b. Embedding Fmoc-Pro on a solid support

The first step in the synthesis of the peptide library was the deposition of Fmoc-Pro on 1g of resin. Prior to attachment of the amino acid derivative, the resin used to react with the following solvents was washed: DMF (dimethylformamide), DCM (methylene chloride) and again DMF, after which the Fmoc shield was removed from the carrier functional group. One Fmoc shield removal cycle included the following steps:

Removal of the Fmoc cover:

20% piperidine in NMP 1 x 3 minutes
20% piperidine in NMP 1 x 8 minutes

Washing
DMF 3 x 2 minutes
IsOH 3 x 2 minutes
DCM 3 x 2 minutes

Chloranil test for the presence of free amino groups.

The resin, with the free amino group, was washed with DMF. In a separate flask, Fmoc-Pro was dissolved in DMF and TBTU, DMAP and finally diisopropylethylamine (DIPEA) were added in the following excess relative to polymer deposition: Fmoc-Pro/TBTU/DMAP/DIPEA, 3:3:2:6. The mixture thus prepared was added to the resin and stirred for 3 hours. The resin was filtered under reduced pressure, washed with DMF, DCM and isopropanol, and the acylation procedure was repeated two times. For the subsequent reaction of Fmoc-Pro attachment to the resin, *O*-(7-azabenzotriazol-1-yl)-*N,N,N',N'*-tetramethyluronium hexafluorophosphate (HATU) was used, and then *O*-(benzotriazol-1-yl)-*N,N,N'N*-tetramethyluronium hexafluorophosphate (HBTU). In the last step, the resin was washed successively with DMF, DCM and isopropanol and dried in the air.

c. Attachment of consecutive protected amino acid residues:

The resin together with the attached Fmoc-Pro residue in the reactor was washed with DMF and then the Fmoc shield from the amine group was deprotected to attach the protected amino acid derivative of glutamic acid.

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

Chloranil test:

As a result of the chloranil test, a positive result was obtained, as evidenced by the green color of the resin grains. This allowed the start of the next stage - attachment of the Fmoc-Glu (OtBu) -OH amino acid residue.

Attachment of an amino acid derivative:

Performed couplings were preceded by washing the resin in DMF. When coupling the protected glutamic acid residue, the composition of the coupling mixture remained unchanged.

At the end of each acylation, a cycle of solvent washes was performed according to the given procedure, followed by a chloranil test for the presence of free amino groups in the solution.

Washing cycle with solvents:

DMF 3 x 2 minutes
IsOH 3 x 2 minutes
DCM 3 x 2 minutes

Chloranil test:

The resin grains during the test after the second acylation were colorless, which allowed for the transition to the next stage of the synthesis, which was the introduction of another protected amino acid derivative - leucine and 2-aminobenzoic acid molecule. The coupling processes were carried out according to the procedure discussed earlier.

Tests carried out after connecting the above-mentioned residues showed positive results, the resin grains were colorless.

d. Removal of the peptide from the carrier with preserving side groups shields

After completion of the synthesis, the protected ABZ-Leu-Glu (OtBu) -Pro-OH peptidewas removed from the carrier together with retaining the side shields with a mixture of acetic acid: TFE (trifluoroethanol):DCM (2:2:6, v/v/v)) in a round bottom flask on a magnetic stirrer.

After 2 hours, the contents of the flask were filtered off under vacuum on Schott sintered frit and washed with astring mixture. The solution was washed with hexane (1:10 v/v), evaporated under reduced pressure and then lyophilized.

e. Chemical synthesis of paranitroanilide derivatives Val

The method of mixed anhydrides was used to synthesize Fmoc-Val-pNA. In the first step, 2 mmol Fmoc-Val was dissolved in anhydrous tetrahydrofuran (THF) in the presence of 2 mmol N-methylmorpholine (NMM). The carboxyl group of the amino acid derivative was activated with 2 mmol of isobutyl chloride. After 10 minutes of activation, 3 mmoles of p-nitroaniline was added. Reactions were carried out for 2 hours at -15°C, followed by a day at room temperature. After the completion of the reaction, the solvent was evaporated and the dry residue was dissolved in ethyl acetate. The resulting solution was washed successively with saturated aqueous NaCl, 10% citric acid, 5% sodium bicarbonate. The solution obtained was dried over anhydrous sodium sulfate, the ethyl acetate was distilled off under reduced pressure, and the dried residue was dried in a vacuum desiccator over $P_2O_5$ and KOH.

f. Coupling of the protected peptide with paranitroanilide Val

The protected ABZ[1]-Leu[2]-Glu (OtBu) [3]-Pro[4]-OH peptide was dissolved in a small amount of DCM, subsequently activated with TFFH (tetramethylfluoroformamide) for 30 minutes at 0 ° C. Then a catalytic amount of DMAP and Fmoc-Val-pNA was added.

The reaction was carried out for 24 hours at room temperature, after which the solvent was evaporated. The resulting solution was quenched with a mixture of astringent side shields: TFA : phenol : water: TIPS (88 : 5 : 5 : 2, v/v/v/v) and stirred in a round bottom flask on a magnetic stirrer for 3 hours.

After this time, cold diethyl ether was added to the flask, and the resulting precipitate was centrifuged on a SIGMA 2K30 centrifuge (Laboratory Centrifuges) within 20 minutes. The precipitate obtained after centrifugation is dissolved in water using ultrasound and then lyophilized.

## Identity / characteristics of a new compound – HPLC analysis, MS

HPLC conditions: RP Bio Wide Pore Supelco C8 column 250 mm 4 mm, phase system A 0.1% TFA in water B: 80% acetonitrile in A), flow 1 ml/min, UV detection at 226 nm. The connection was confirmed.

Example 3

Study of new compounds obtained according to example 1 and 2 for use in the diagnosis of inflammation and diagnosis of epithelial neoplasms, in particular tumors

The study was performed on a group of 130 patients - diagnosed epithelial neoplasms and/or inflammation, 430 people without neoplasms, cancer/.inflammation in the form of control.

Incubation of the ABZ-Leu-Glu-Pro-Val-ANB-NH$_2$ / ABZ-Leu-Glu-Pro-Val-pNA compound in the conditions described below.

- 80 $\mu$l of urine of patients with a diagnosis of urinary tract epithelium or urine from the control group
- 100 $\mu$l buffer (50 mM Tris-HCl, pH 8.3)
- 20 $\mu$l of ABZ-Leu-Glu-Pro-Val-ANB NH$_2$-NH$_2$ substrate (0.5 mg/ml DMSO - dimethylsulfoxide) or ABZ-Leu-Glu-Pro-Val-pNA (0.5 mg/ml DMSO - dimethyl sulfoxide)
- The incubation time is 60 minutes
- Temperature 37°C

A study was also carried out on the use of the diagnostic marker obtained according to example 1 or 2 for the detection of inflammation. For this purpose, the marker was added to the urine with a diagnosed inflammation, confirmed by the detection of a marker of inflammation such as neutrophil elastase. As a result of the research, it was shown that this marker can be used as a marker of inflammation, as well as a marker of inflammation and epithelial neoplasms, cancer, in the presence of neoplasms/cancer and confirmed inflammation. In both cases, the color was observed - absorbance reading at a wavelength around 410 nm - in the 380-430 nm range.

The following results were obtained by incubation, examples of which are shown and described below.

Studies on the use of these compounds to detect inflammation and epithelial neoplasms, tumors in blood samples of patients with known inflammation/epithelial neoplasms/tumors were also conducted. The process of absorbance measurement is carried out in the same way as described for urine samples. Initial test results confirm their diagnostic use also for this biological material - plasma or serum.

HPLC analysis:

Substrate ABZ-Leu-Glu-Pro-Val-ANB $NH_2$ in a buffer with 50 mM Tris-HCl, pH 8.3

ABZ-Leu-Glu-Pro-Val-ANB-NH$_2$, $t_R$=22.89

Hydrolysis of the ABZ-Leu-Glu-Pro-Val-ANB $NH_2$ substrate in urine with diagnosed neoplasm, in particular cancer of the urinary epithelium

Urine sample with substrate

ABZ-Leu-Glu-Pro-Val-OH $t_R$=19.56

ABZ-Leu-Glu-Pro-Val-ANB-NH$_2$, $t_R$=19.89

The proteolytic activity of the enzymes present in the examined biological material (urine samples with diagnosed neoplasm - e.g. cancer) is proportional to the intensity of the observed absorbance signal released by the hydrolysis of the ANB peptide bond. The analysis confirmed that in the case of the tested samples, substrate hydrolysis of $ABZ^1$-$Leu^2$-$Glu^3$-$Pro^4$-$Val^5$-$ANB$-$NH_2^6$ takes place at position 5, where we obtain $ABZ^1$-$Leu^2$-$Glu^3$-$Pro^4$-$Val^5$ and $ANB$-$NH_2^6$. An analogous process is the compound $ABZ^1$-$Leu^2$-$Glu^3$-$Pro^4$-$Val^5$-$pNA^6$ which releases the free molecule pNA and the fragment $ABZ^1$-$Leu^2$-$Glu^3$-$Pro^4$-$Val^5$-$OH$.

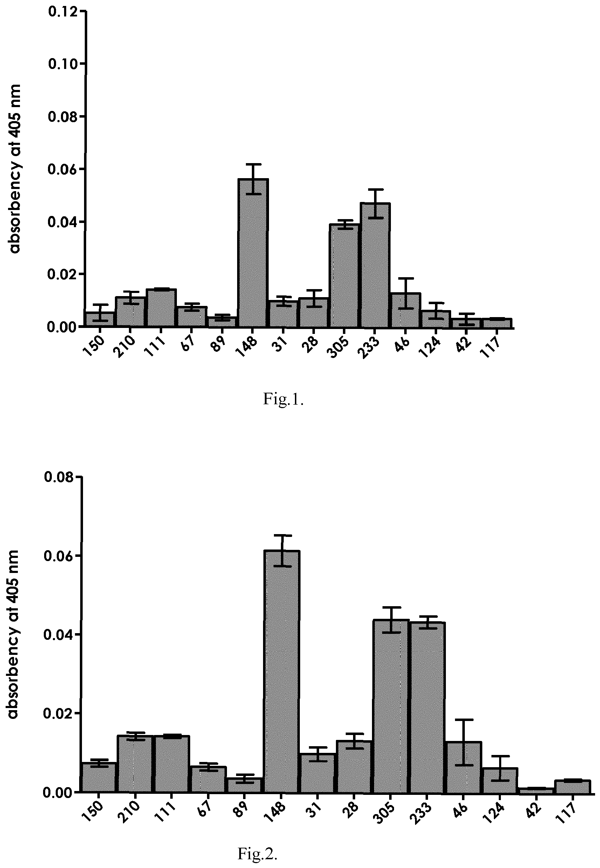

From the graph - Fig. 1, we can read that in the case of samples 148, 305 and 233 the degradation of the ABZ-Leu-Glu-Pro-Val-ANB-$NH_2$ substrate takes place more efficiently than for materials 89, 42 or 117. This result may be due to the difference in activity and the amount of enzymes responsible for proteolysis. The same image is obtained using the ABZ-Leu-Glu-Pro-Val-pNA substrate - Fig.2.

Table 1.

| Types of inhibitors, the mechanism of their action and the effective concentration used | | | |
|---|---|---|---|
| Inhibitor | Type of inhibitor | Class braked of enzymes | Concentration [M] |
| Carfilzomib | irreversible | threonine proteases (chymotrypsin activity of the human roteasome 20S) | $1,000 \times 10^{-8}$ |
| AHX-VYDnV$^P$ $(C_4H_2Cl)_2$ | irreversible | serine proteinases | $4,625 \times 10^{-4}$ |
| Bt-NI(OBzl)YDA$^P$ $(C_4H_2Cl)_2$ | irreversible | serine proteinases | $3,215 \times 10^{-4}$ |
| E-64 | irreversible | cysteine proteinases | $3,495 \times 10^{-4}$ |

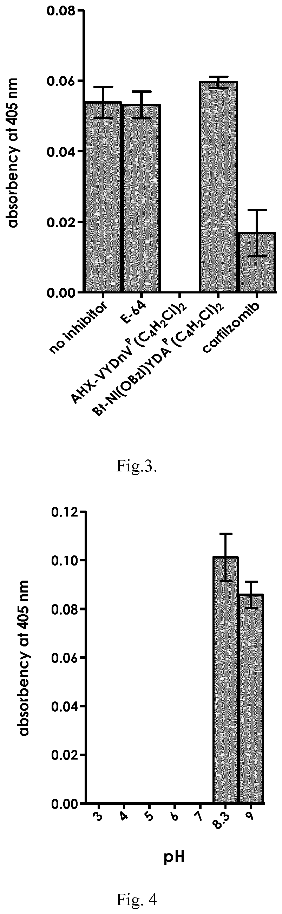

Proteolytic activity is dependent on the presence of specific compounds called inhibitors (proteolytic enzymes). Their incubation with a given biological material allows us to pre-determine the class of enzymes present in the tested samples. For this purpose, we have added effective inhibitory concentrations of certain classes of proteolytic enzymes to the combined urine samples of the ill persons - patients (urothelial epithelial neoplasms, in particular tumor). The result of this experiment shows figure 3.

As a result of this experiment, it was found that there are at least two enzymes in the test material exhibiting two maximum activity at the acidic and basic pH.

The use of inhibitors of different classes of enzymes did not result in a decrease in proteolytic activity. Only incubation of urine with AHX-VYDnV$^P$ $(C_4H_2Cl)_2$ resulted in a complete inhibition of hydrolysis. Such a result could suggest the presence of serine proteinases, more specifically those with elastase specificity. We made an additional experiment that confirmed that the compound we use ABZ-Leu-Glu-Pro-Val-ANB-$NH_2$ is hydrolyzed by elastase, while proteinase 3 does not cause proteolysis. The observed activity may be indicative of an inflammatory condition associated with cancer, as elastase is released by neutrophils as an immune response to inflammation. Carfizomib also inhibits proteolysis to some extent, hence the conclusion that the enzymatic activity in the urine of people diagnosed with bladder neoplasms, in particular cancer is caused by the enzyme (s) belonging to a different type from that listed in the table above (fig. 3).

Many factors affect the enzymatic activity, one of which is the pH of the reaction medium. The conducted experiment may indicate that enzymes that prefer different conditions are found in the tested urine. We observe one maximum of proteolytic activity, which suggests the presence of proteinases that hydrolyze under basic conditions (fig. 4).

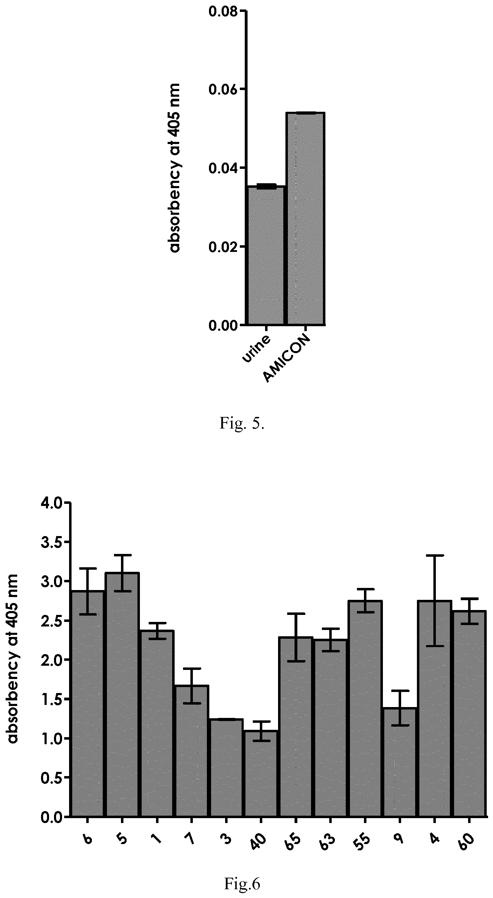

To increase the sensitivity of the detection method, the obtained biological material (human urine with a confirmed urinary tract epithelium) was thickened. For this purpose, Amicon filter columns were used. As a result of this process, we obtained a four times folded material with activity about 2 times higher than the starting material (fig. 5). The fact of a linear response to the increasing concentration of the enzyme confirms the selectivity of the obtained compounds.

Summary

The analysis confirmed that the compounds of example 1 and 2 are for use in the *in vitro* diagnosis - their use in the diagnosis of inflammation and in the diagnosis of epithelial neoplasms, in particular cancer. Preliminary results indicate that these compounds may be used to identify inflammation - an immune response that accompanies epithelial neoplasm, cancer as well as to diagnose an additional inflammatory condition e.g. caused by an infection that accompanies neoplasm or in the absence of neoplasms, e.g. cancer, i.e. the recognition of the immune response itself, which, e.g., can lead to a disease process including neoplasm, in particular cancer.

The mechanism of action of the new compounds - diagnostic markers - according to the invention is based on enzymatic hydrolysis at a peptide site, thereby liberating the ANB-NH$_2$-5-amino-2-nitrobenzoic acid amide or pNA, which has an absorbance at a wavelength of 300-500 nm, especially 380-430 nm.

Example 4

Synthesis of the compound: ABZ[1]-Met[2]-Lys[3]-Val[4]-Trp[5]-ANB-NH$_2$[6]

The process is carried out analogously to example 1, using appropriate other amino acid derivatives than in example 1.

a) The first step in the synthesis was to obtain a chromogenic peptide which was obtained by solid phase synthesis using Fmoc/tBu chemistry.

A compound of the sequence ABZ[1]-Met[2]-Lys[3]-Val[4]-Trp[5]-ANB-NH$_2$[6], where ABZ is 2-amino benzoic acid and ANB-NH$_2$ is 5-amino-2-benzoic acid amide, and ANB is 5-amino-2-benzoic acid obtained by solid phase chemical synthesis using the following amino acid derivatives.
Boc-ABZ, Fmoc-Met, Fmoc-Lys(Boc), Fmoc-Val, Fmoc-Trp(tBu), ANB
The synthesis of peptide substrates was carried out on a solid support:

- TentaGel S RAM resin from RAPP Polymere (Germany) with a deposition of 0.23 mmol/g

The synthesis of all peptides was carried out manually using a laboratory shaker. For most of the stages, the reactors were a syringe ended with sinter for synthesis in a solid phase with a capacity of 25 ml.
All synthesized substrates contained in their sequence at the N-terminus 2-aminobenzoic acid (ABZ) and a 5-amino-2-nitrobenzoic C-terminal molecule. ABZ acted as a fluorescence donor, whereas ABZ acted as a fluorescence quencher and at the same time a chromophore. The peptides in their sequence contained one reactive site located between the amino acid residues Trp-ANB-NH$_2$
Deposition of ANB on TentaGel S RAM resin:
The peptide synthesis was performed on a Rapp Polymere resin TentaGel S RAM with a deposition of 0.23 mmol/g. In the first stage, the resin was loosened through a series of washes. Subsequent removal of the amine Fmoc protecting group from the carrier was carried out with a 20% piperidine solution in NMP. Then a cycle of solvent washes was carried out. In order to confirm the presence of free amino groups, a chloranil test was carried out. Wash cycle with solvents: DMF 1 x 10 minutes.

IsOH 1 x 10 minutes
DCM 1 x 10 minutes. Removal of Fmoc: DMF 1 x 5 minutes
20% piperidine in NMP 1 x 3 minutes
20% piperidine in NMP 1 x 8 minutes
Wash cycle with solvents: DMF 3 x 2 minutes
IsOH 3 x 2 minutes
DCM 3 x 2 minutes Chloranil test:

The chloranil test consisted of transferring, by means of a spatula, several resin beads from the reactor - a syringe into a glass ampoule, to which was then added 100 μl of a saturated solution of p-chloranil in toluene and 50 μl of fresh acetaldehyde. After 10 minutes, the color control of the grains was carried out.
At this stage, after the test, green color of the grains was obtained, which indicated the presence of free amine groups. After confirming the removal of the fluorenylmethoxycarbonyl guards from the resin, the next step could be taken to attach the ANB derivative (5-amino-2-nitrobenose acid)

Deposition of 5-amino-2-nitrobenzoic acid on a solid support

The first step in the synthesis of the peptide library was the deposition of ANB per 1g of resin. Prior to attaching the chromophore, the resin used for the reaction was washed with the following solvents: DMF, DCM and again DMF, after which the Fmoc shield was removed from the carrier functional group. One Fmoc shield removal cycle included the following steps:

1. Removal of the Fmoc cover:

A 20% piperidine solution in NMP 1 × 3 minutes
1 × 8 minutes

2. Washing

DMF 3 × 2 minutes
isopropanol 3 × 2 minutes
DCM 3 × 2 minutes

3. Test for the presence of free amino groups: Chloranilowy [167].

The amine free resin was washed with 5% N-methylmorpholine (NMM) in DMF followed by DMF. The Fmoc shield removal procedure and the wash cycle were performed in a Merrifield dish. ANB was dissolved in DMF in a separate flask and TBTU, DMAP and finally diisopropylethylamine (DIPEA) were added in the following excess to the polymer deposition: ANB/TBTU/DMAP/DIPEA, 3: 3: 2: 6. The mixture thus prepared was added to the resin and stirred for 3 hours. The resin was filtered under reduced pressure, washed with DMF, DCM and isopropanol, and the acylation procedure was repeated two times. To carry out subsequent ANB-attachment reactions, $O$-(7-azabenzotriazol-1-yl)-$N,N,N',N'$-tetramethyluronium hexafluorophosphate (HATU) was used for the resin, and then $O$-(benzotriazol-l-yl)-$N,N,N'N$-tetramethyluronium hexafluorophosphate (HBTU). In the last step, the resin was washed successively with DMF, DCM and isopropanol and dried in the air.

Attachment of the C-terminal amino acid residue to the ANB

The corresponding amino acid derivative (9-fold molar excess relative to the resin deposition) was dissolved in pyridine and transferred to a flask containing ANB embedded resin. The whole was cooled to -15°C (ice bath: 1 part $NH_4Cl$ weight, 1 part $NaNO_3$ weight, 1 part ice weight). After reaching the desired temperature, $POCl_3$ (in a 1:1 ratio to the amount of amino acid derivative used) was added and the mixture was stirred on a magnetic stirrer: 20 minutes at -15°C, 30 minutes at room temperature, and 6 hours at 40°C (oil bath). After completion of the reaction, the resin was filtered under reduced pressure, washed with DMF and MeOH and allowed to dry.

In the next stage, the rest was attached to the P2 position (serine)

All attachment of amino acid residues was preceded by washing the resin with DMF for 5 minutes. Diisopropylcarbodiimide was used as coupling agent in the subsequent attachments. The procedure was repeated twice.

After each acylation, a resin wash cycle was started, followed by a chloranil test to monitor the attachment of the amino acid derivative to the free amino groups of the resin.

Wash cycle with solvents: DMF 3 x 2 minutes
IsOH 3 x 2 minutes
DCM 3 x 2 minutes Chloranil test:

As a result of the tests carried out after the first two couplings, the grain color was first green and then gray, so it was necessary to perform another acylation, as a result of which the resin grains tested with the chloranil test were colorless. This showed the attachment of ANB to the TentaGel S RAM resin, which allowed the transition to the next stage of peptide synthesis.

Attachment of consecutive protected amino acid residues:
The resin together with the attached ANB residue in the reactor was washed with DMF and then the Fmoc shield from the amine group was deprotected to attach the protected amino acid derivative of glycine.

Cover removal of Fmoc: DMF 1 x 5 minutes
20% piperidine in NMP 1 x 3 minutes
20% piperidine in NMP 1 x 8 minutes
Wash cycle with solvents: DMF 3 x 2 minutes
IsOH 3 x 2 minutes
DCM 3 x 2 minutes Chloranil test:

As a result of the chloranil test, a positive result was obtained, as evidenced by the green color of the resin grains. This allowed the start of the next stage - attachment of the Fmoc-Trp-OH amino acid residue.

Attachment of an amino acid derivative:
Performed couplings were preceded by washing the resin in DMF. When coupling the protected glycine residue, the composition of the coupling mixture remained unchanged.

At the end of each acylation, a cycle of solvent washes was performed according to the given procedure, followed by a chloranil test for the presence of free amino groups in the solution.

Wash cycle with solvents: DMF 3 x 2 minutes
IsOH 3 x 2 minutes
DCM 3 x 2 minutes Chloranil test:

Grains of the test resin carried out after the second acylation were colorless, which allowed for the transition to the next stage of the synthesis, which was the introduction of further protected amino acid derivatives - valine, lysine, methionine and the 2-aminobenzoic acid molecule. The coupling processes were carried out according to the procedure discussed earlier.

Tests carried out after connecting the above-mentioned residues showed positive results, the resin grains were colorless.

b) Removal of the peptide from the carrier

After completion of the synthesis, the peptide amide was removed from the support with the simultaneous removal of the side shields by means of a mixture of: TFA : phenol: water: TIPS (88 : 5 : 5 : 2, v/v/v/v) in a round bottom flask on a magnetic stirrer.

After 3 hours, the contents of the flask were filtered under vacuum on Schott sintered frit and washed with diethyl ether. The resulting precipitate was centrifuged on a SIGMA 2K30 centrifuge (Laboratory Centrifuges) for 20 minutes. The precipitate obtained after centrifugation is dissolved in water using ultrasound and then lyophilized.

c) Identity/characteristics of the new compound - HPLC, MS and NMR analysis

HPLC conditions: RP Bio Wide Pore Supelco C8 column 250 mm 4 mm, phase system A 0.1%
TFA in water B: 80% acetonitrile in A), flow 1 ml/min, UV detection at 226 nm.
tR 20.1 MS MALDI TOF matrix alpha cinnamon acid molecular weight 844.4 Da calculated 844,4.

Example 5

Confirmation of the use of the compound of example 4 in the diagnosis of epithelial neoplasms, cancer.

The process was carried out similarly as described in example 3, except that a different compound was used in the diagnosis of epithelial neoplasm, cancer. Incubation of this compound in the conditions described below.

- 3 $\mu$l of urine of patients diagnosed with urinary tract epithelium neoplasms or urine from a healthy patient
- 177 $\mu$l buffer (50 mM Tris-HCl, pH 8.3)
- 20 $\mu$l of ABZ-Met-Lys-Val-Trp-ANB-NH2 substrate (0,5 mg/ml)
- time 60 minutes
- Temperature 37°C

It gives the results which are shown below.

HPLC analysis:

| Substrate<br>ABZ-Met-Lys-Val-Trp-ANB-NH$_2$ | Substrate hydrolysis<br>of ABZ-Met-Lys-Val-Trp-ANB-NH$_2$ |
|---|---|
| in buffer | in urine with diagnosed neoplasms, in particular cancer of the urinary epithelium |
| | |

| Sample with bortezomib | |
|---|---|
| in the urine with diagnosed neoplasms e.g. cancer of the urinary tract | healthy urine |
| | |

The proteolytic activity of the enzymes present in the examined biological material (urine samples with diagnosed cancer) is proportional to the intensity of the observed absorbance signal released by the hydrolysis of the ANB peptide bond. The substrate hydrolysis of ABZ[1]-Met[2]-Lys[3]-Val[4]-Trp[5]-ANB-NH$_2$[6] is at the 5-position where we obtain ABZ[1]-Met[2]-Lys[3]-Val[4]-Trp[5] and ANB-NH$_2$[6].

The peptide (substrate) with the sequence ABZ[1]-Met[2]-Lys[3]-Val[4]-Trp[5]-ANB-NH$_2$[6] appears at low absorbance at 405/410 nm. During the hydrolysis, a free ANB-NH$_2$ molecule is released which has a maximum absorbance at 405/410. In other words, peptide hydrolysis causes an increase in ANB-NH$_2$ concentration and thus an increase in absorbance at 405/410 nm.

From the graph (Fig.6), it can be read that in the case of samples 5, 6, 4 and 60, the degradation of the ABZ-Met-Lys-Val-Trp-ANB-NH$_2$ substrate takes place more efficiently than in the materials 40, 3, 9 or 7. This result may be due to the difference in activity and the amount of enzymes responsible for proteolysis.

Table 2

| Inhibitor | Type | The class of inhibited enzymes | Concentration final in the measuring well [M] |
|---|---|---|---|
| Carfilzomib | Irreversible | threonine proteases (chymotrypsin activity of human proteasome 20S) | $1,000 \times 10^{-8}$ |

(continued)

| Inhibitor | Type | The class of inhibited enzymes | Concentration final in the measuring well [M] |
|---|---|---|---|
| Bestatin | irreversible | aminopeptidase | $3,625 \times 10^{-4}$ |
| AEBSF | irreversible | serine proteinases | $5,215 \times 10^{-4}$ |
| E-64 | irreversible | cysteine proteinases | $3,495 \times 10^{-4}$ |
| Leupeptin | reversible | serine proteinases and cysteine | $2,630 \times 10^{-4}$ |

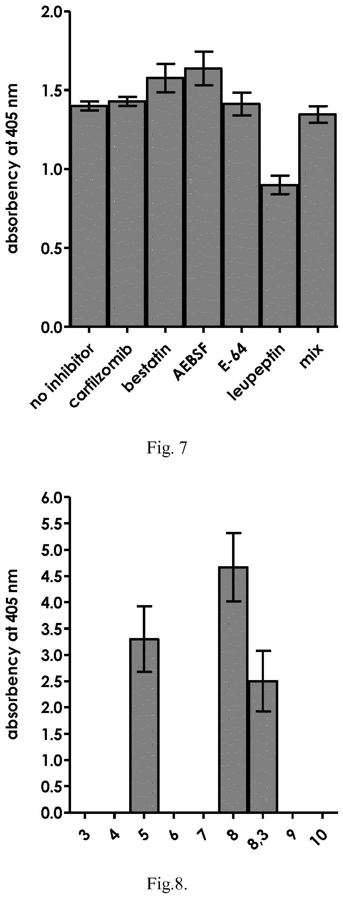

Proteolytic activity is dependent on the presence of certain compounds called inhibitors for this purpose in combined urine samples of patients (urothelial carcinoma), we added effective inhibitory concentrations of certain classes of proteolytic enzymes. The result of this experiment is shown in fig. 7.

As a result of this experiment, it was found that there are at least two enzymes in the test material exhibiting two maximum activity at acidic and basic pH.

The use of inhibitors of different classes of enzymes did not result in a decrease in proteolytic activity. Only incubation of urine with leupeptin resulted in a decrease in the hydrolysis efficiency. Such a result could suggest the presence of serine or cysteine proteinases. Other used inhibitors also inhibit these classes of enzymes, hence the conclusion that enzymatic activity in the urine of people diagnosed with bladder cancer is due to the enzyme (s) belonging to a different type from those listed in the table above (Fig. 7).

Many factors affect the enzymatic activity, one of which is the pH of the reaction medium. The conducted experiment may indicate that enzymes that prefer different conditions are found in the tested urine. We observe two maxima of proteolytic activity, suggesting the presence of at least two proteinases - at least one of which hydrolyzes under acidic conditions and the other alkaline (fig. 8).

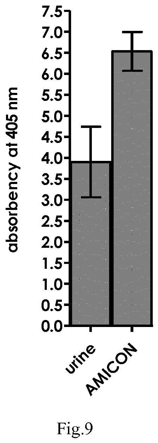

To increase the sensitivity of the detection method, the obtained biological material (human urine with a confirmed urinary tract epithelium) was thickened. For this purpose, Amicon filter columns were used. As a result of this process, we obtained a four-fold thick material with activity about 4 times higher than the starting material (fig. 9). The fact of a linear response to the increasing concentration of the enzyme confirms the selectivity of the resulting compound.

Example 6

Preparation of compound $ABZ^1$-$Met^2$-$Lys^3$-$Val^4$-$Trp^5$-$pNA^6$ where:

ABZ means 2-aminobenzoic acid,
pNA means 4-nitroaniline.

The process is carried out analogously to example 2 and similar to example 1, using appropriate other amino acid derivatives than in example 2 and 1.

Obtaining p-nitroanilide Trp

a. The first step in the synthesis was to obtain a protected peptide that was obtained by solid phase synthesis using Fmoc/tBu chemistry.

Compound $ABZ^1$-$Met^2$-$Lys(Boc)^3$-$Val^4$-OH, where ABZ is 2-aminobenzoic acid, obtained by solid phase chemical synthesis using the following amino acid derivatives:
Boc-ABZ, Fmoc-Met, Fmoc-Lys(Boc), Fmoc-Val.

The synthesis of the compound was carried out on a solid support:

- 2-chloro-chlorotinyl resin, e.g. from Iris BIOTECH GMBH (Germany) with deposition of 1.6 mmol Cl/g groups

The synthesis of the compound was carried out manually using a laboratory shaker. A syringe ended with sinter for solid-phase synthesis with a capacity of 25 ml. was the reactor by all stages.

The peptide synthesis was performed on a 2-chloro-chlorotinyl resin from Iris BIOTECH GMBH (Germany) with a deposition of 1.6 mmol Cl /g. In the first stage, the resin was loosened through a series of washes. Subsequent removal of the amine Fmoc protecting group from the carrier was carried out with a 20% piperidine solution in NMP. Then a cycle of solvent washes was carried out. In order to confirm the presence of free amino groups, a chloranil test was carried out.

Washing cycle with solvents:

DMF 1 x 10 minutes
IsOH 1 x 10 minutes
DCM 1 x 10 minutes

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

b) Chloranil test:

The chloranil test consisted of transferring, by means of a spatula, several resin beads from the reactor - a syringe into a glass ampoule, to which was then added 100 $\mu$l of a saturated solution of p-chloranil in toluene and 50 $\mu$l of fresh acetaldehyde. After 10 minutes, the color control of the grains was carried out.

At this stage, after the test, green color of the grains was obtained, which indicated the presence of free amine groups. After confirming the removal of the 9-fluorenylmethoxycarbonyl protections from the resin, it was possible to proceed to the next step of attaching the Fmoc-Val derivative.

b. Embedding Fmoc-Val on a solid support

The first step in the synthesis of the peptide library was the deposition of Fmoc-Val on 1g of resin. Prior to attachment of the amino acid derivative, the resin used to react with the following solvents was washed: DMF (dimethylformamide), DCM (methylene chloride) and again DMF, after which the Fmoc shield was removed from the carrier functional group. One Fmoc shield removal cycle included the following steps:

Removal of the Fmoc cover:

20% piperidine in NMP 1 x 3 minutes
20% piperidine in NMP 1 x 8 minutes
Washing
DMF 3 x 2 minutes
IsOH 3 x 2 minutes
DCM 3 x 2 minutes
Chloranil test for the presence of free amino groups.

The resin, with the free amino group, was washed with DMF. In a separate flask, Fmoc-Pro was dissolved in DMF and TBTU, DMAP and finally diisopropylethylamine (DIPEA) were added in the following excess relative to polymer deposition: Fmoc-Pro/TBTU/DMAP/DIPEA, 3:3:2:6. The mixture thus prepared was added to the resin and stirred for 3 hours. The resin was filtered under reduced pressure, washed with DMF, DCM and isopropanol, and the acylation procedure was repeated two times. For the subsequent reaction of Fmoc-Pro attachment to the resin, *O*-(7-azabenzotriazol-1-yl)-*N,N,N',N'*-tetramethyluronium hexafluorophosphate (HATU) was used, and then *O*-(benzotriazol-1-yl)-*N,N,N'N'*-tetramethyluronium hexafluorophosphate (HBTU). In the last step, the resin was washed successively with DMF, DCM and isopropanol and dried in the air.

c. Attachment of consecutive protected amino acid residues:

The resin together with the attached Fmoc-Val residue in the reactor was washed with DMF and then the Fmoc shield from the amine group was deprotected to attach the protected amino acid derivative of glutamic acid.

Removal of the Fmoc cover:

DMF 1 x 5 minutes
20% piperidine in NMP 1 x 3 minutes
20% piperidine in NMP 1 x 8 minutes

Washing cycle with solvents:

DMF 3 x 2 minutes
IsOH 3 x 2 minutes
DCM 3 x 2 minutes

Chloranil test:

As a result of the chloranil test, a positive result was obtained, as evidenced by the green color of the resin grains. This allowed the start of the next stage - attachment of the Fmoc-Lys (Boc)-OH amino acid residue.
Attachment of an amino acid derivative:
Performed couplings were preceded by washing the resin in DMF. When coupling the protected glutamic acid residue, the composition of the coupling mixture remained unchanged.
At the end of each acylation, a cycle of solvent washes was performed according to the given procedure, followed by a chloranil test for the presence of free amino groups in the solution.
Washing cycle with solvents:

DMF 3 x 2 minutes
IsOH 3 x 2 minutes
DCM 3 x 2 minutes

Chloranil test:

The resin grains during the test after the second acylation were colorless, which allowed for the transition to the next stage of the synthesis, which was the introduction of another protected amino acid derivative - methionine and 2-aminobenzoic acid molecule. The coupling processes were carried out according to the procedure discussed earlier.
Tests carried out after connecting the above-mentioned residues showed positive results, the resin grains were colorless.

d. Removal of the peptide from the carrier with preserving side groups shields

After completion of the synthesis, the protected ABZ-Met-Lys (Boc)-Val-OH peptide was removed from the carrier together with retaining the side shields with a mixture of: acetic acid: TFE (trifluoroethanol):DCM (2:2:6, v/v/v)) in a round bottom flask on a magnetic stirrer.
After 2 hours, the contents of the flask were filtered off under vacuum on Schott sintered frit and washed with astring mixture. The solution was washed with hexane (1:10 v/v), evaporated under reduced pressure and then lyophilized.

e. Chemical synthesis of paranitroanilide derivatives Val

The method of mixed anhydrides was used to synthesize Fmoc-Trp-pNA. In the first step, 2 mmol Fmoc-Trp was dissolved in anhydrous tetrahydrofuran (THF) in the presence of 2 mmol N-methylmorpholine (NMM). The carboxyl group of the amino acid derivative was activated with 2 mmol of isobutyl chloride. After 10 minutes of activation, 3 mmoles of p-nitroaniline was added. Reactions were carried out for 2 hours at -15°C, followed by a day at room temperature. After the completion of the reaction, the solvent was evaporated and the dry residue was dissolved in ethyl acetate. The resulting solution was washed successively with saturated aqueous NaCl, 10% citric acid, 5% sodium bicarbonate. The solution obtained was dried over anhydrous sodium sulfate, the ethyl acetate was distilled off under reduced pressure, and the dried residue was dried in a vacuum desiccator over $P_2O_5$ and KOH.

f. Coupling of the protected peptide with paranitroanilide Trp

The protected ABZ$^1$-Met$^2$-Lys (Boc)$^3$-Val$^4$-OH peptide was dissolved in a small amount of DCM, subsequently activated with TFFH (tetramethylfluoroformamide) for 30 minutes at 0°C. Then a catalytic amount of DMAP and Fmoc-Trp-pNA was added. The reaction was carried out for 24 hours at room temperature, after which the solvent was evaporated. The resulting solution was quenched with a mixture of astringent side shields: TFA : phenol : water: TIPS (88 : 5 : 5 : 2, v/v/v/v) and stirred in a round bottom flask on a magnetic stirrer for 3 hours.

After this time, cold diethyl ether was added to the flask, and the resulting precipitate was centrifuged on a SIGMA 2K30 centrifuge (Laboratory Centrifuges) within 20 minutes. The precipitate obtained after centrifugation is dissolved in water using ultrasound and then lyophilized.

Identity / characteristics of a new compound - HPLC analysis, MS

HPLC conditions: RP Bio Wide Pore Supelco C8 column 250 mm 4 mm, phase system A 0.1% TFA in water B: 80% acetonitrile in A), flow 1 ml/min, UV detection at 226 nm. The connection was confirmed.

Example 7

Use of the compound obtained according to example 4 for the in vitro diagnosis of epithelial neoplasm, in particular tumor in biological samples.

The ABZ-Met-Lys-Val-Trp-pNA compound was analyzed for use in the diagnosis of epithelial neoplasm similar to that described in example 3. The analysis was carried out by assessing the protolytic activity of human urine in samples of ill persons: with confirmed epithelial neoplasms, in particular cancer and in the control group: without inflammation and cancer.

Procedure

80 $\mu$l of urine of patients diagnosed with urinary tract epithelium or urine from a healthy patient

- 100 $\mu$l buffer (50 mM Tris-HCl, pH 8.3)
- 20 $\mu$l of ABZ-Leu-Glu-Pro-Val-ANB NH$_2$-NH$_2$ substrate (0.5 mg/ml DMSO - dimethylsulfoxide) or ABZ-Leu-Glu-Pro-Val-pNA (0.5 mg/ml DMSO - dimethyl sulfoxide)
- Temperature 37°C
- Measurement of absorbance increase was monitored in the 380-430 nm range for 60 minutes.

As a result of the incubation, the increase in the absorbance in the measurable range for the samples of patients was registered appropriately, no increase in the absorption for healthy samples, as shown in fig. 1 and fig. 2 and as described in example 3. Preliminary results indicate that the compounds according to example 1 and 2 allow the detection of an inflammatory response accompanied by secretion of proteolytic enzymes present in the examined biological material and this immunological process may accompany the epithelial tumor. In the case of the compounds of example 4, preliminary results indicate that they enable the diagnosis of epithelial neoplasms, in particular tumor.

Example 8

Diagnosis of inflammation and epithelial neoplasms, in particular tumors - a diagnostic kit.

The diagnostic kit contains at least two compounds: markers which are described in the above examples, in that the kit contains at least one compound A - a marker of inflammation and epithelial neoplasm with the general formula ABZ$^1$-Leu$^2$-Glu$^3$-Pro$^4$-Val$^5$-X$^6$ and compound B - epithelial neoplasm marker of the formula ABZ$^1$-Met$^2$-Lys$^3$-Val$^4$-Trp$^5$-X, where: ABZ is 2-aminobenzoic acid, X is ANB-NH$_2$ or pNA, where ANB-NH2 is 5-amino-2-benzoic acid amide, pNA means p-nitroaniline. These compounds undergo hydrolysis under the influence of proteolytic enzymes - markers of inflammation and/or cancer. Because of the coexistence of the neoplastic process and the immune response elicited above (inflammation), two types of developed compounds were used in the differential diagnosis of these two states.

Compounds were used in the following procedure:

- up to 80 $\mu$l of urine of patients with a diagnosis of urinary tract epithelium neoplasm/inflammation or urine from a healthy patient
- 100 $\mu$l buffer (50 mM Tris-HCl, pH 8.3)
- 20 $\mu$l of substrate - respectively labeled marker A and B added separately to another sample of the same biological material - compound in buffer 0.5 mg/ml DMSO - dimethyl sulfoxide

- Temperature 37°C
- The measurement of the increase in absorbance was monitored in the 380-430 nm range, especially 410 nm.

The increase in absorbance using compound A in the range described above in samples with only inflammation and both inflammation and confirmed epithelial neoplasm indicates the presence of an enzyme of human neutrophils closely related to inflammation. Their presence is also associated with the immune response induced in some of the test samples by the presence of neoplasm cells.

Experimental studies also indicate that in the absence of coloration - hydrolysis of the added marker B - in the sample of biological material and the presence of absorbance in the same sample in the presence of marker A, it is possible to diagnose the presence

of inflammation, for example bacterial or viral infection. Preliminary results indicate that the compounds of the invention have many applications in the diagnosis of immunological processes and especially epithelial neoplasms.

**Claims**

1. Compound defined by general formula: $ABZ^1$-$Leu^2$-$Glu^3$-$Pro^4$-$Val^5$-$X^6$, where: ABZ denotes 2-aminobenzoic acid, X denotes ANB-$NH_2$ or pNA, wherein ANB-$NH_2$ denotes 5-amino-2-benzoic acid, pNA denotes 4-nitroaniline.

2. Compound defined by general formula: $ABZ^1$-$Met^2$-$Lys^3$-$Val^4$-$Trp^5$-$X^6$, where: ABZ denotes 2-aminobenzoic acid, X denotes ANB-$NH_2$ or pNA, wherein ANB-$NH_2$ is 5-amino-2-benzoic acid, pNA is 4-nitroaniline.

3. Method for synthesis of compound defined by formula $ABZ^1$-$Leu^2$-$Glu^3$-$Pro^4$-$Val^5$-$ANB$-$NH_2^6$ wherein: ABZ denotes 2-aminobenzoic acid, ANB-$NH_2$ denotes 5-amino-2-benzoic acid amide, **characterized in that**, the compound is obtained in a solid support synthesis and the process is carried out in the following steps:

   a) preparation of a resin enabling the conversion of an acid into an amide,
   b) deprotection of the protective group,
   c) deposition of 5-amino-2-nitrobenzoic acid ANB,
   d) repeating steps from b) to c) until a compound is obtained, wherein the synthesis is carried out from residues 6 to 1, followed by cleavage of the obtained peptide from the resin.

4. Method for synthesis of compound defined by the formula $ABZ^1$-$Leu^2$-$Glu^3$-$Pro^4$-$Val^5$-$pNA^6$ wherein ABZ denotes 2-aminobenzoic acid, pNA denotes 4-nitroaniline, **characterized in that**, the compound is obtained in the synthesis on a solid support and the method is carried out in the following steps:

   a) preparation of 2-chloro-chlorotrityl resin,
   b) deprotection of the protective group,
   c) embedding of Pro4 on the resin,
   d) repeating steps from b) to c) until obtaining ABZ-Leu-Glu-Pro;
   e) cleavage of the obtained peptide from the resin
   f) obtaining the amino acid paranitroanilide Val-pNA
   g) coupling of paranitroanilide with a protected peptide ABZ-Leu-Glu-Pro + Val-pNA,
   h) removing the side groups of amino acids.

5. Method for synthesis of compound defined by the formula: $ABZ^1$-$Met^2$-$Lys^3$-$Val^4$-$Trp^5$-$ANB$-$NH_2^6$ wherein: ABZ denotes 2-aminobenzoic acid, ANB-$NH_2$ denotes 5-amino-2-benzoic acid amide, **characterized in that**, the compound is obtained in a solid support synthesis and the process is carried out in the following steps:

   a) preparation of a resin enabling the conversion of an acid into an amide,
   b) deprotection of the protective group,
   c) deposition of 5-amino-2-nitrobenzoic acid ANB,
   d) repeating steps from b) to c) until a compound is obtained, wherein the synthesis is carried out from residues 6 to 1, followed by cleavage of the obtained peptide from the resin.

6. Method for synthesis of compound defined by general formula $ABZ^1$-$Met^2$-$Lys^3$-$Val^4$-$Trp^5$-$pNA^6$, wherein ABZ denotes 2-aminobenzoic acid and pNA denotes 4-nitroaniline, **characterized in that** the compound is obtained in the synthesis on a solid support and the method is carried out in the following steps:

    a) preparation of 2-chlorochlorotrythyl resin,
    b) deprotection of the protective group,
    c) embedding Val4 on the resin,
    d) repeating steps b) to c) until obtaining $ABZ^1$-$Met^2$-$Lys^3$-$Val^4$,
    e) cleaving off the obtained peptide from the resin,
    f) obtaining p nitroanilide of the amino acid Trp-pNA,
    g) coupling of paranitroanilide with a protected peptide (ABZ-Leu-Glu-Pro + Trp-pNA),
    h) removing the side groups of amino acids.

7.  Diagnostic marker defined by the general formula $ABZ^1$-$Leu^2$-$Glu^3$-$Pro^4$-$Val^5$-$X^6$ where: ABZ denotes 2-aminobenzoic acid, X denotes ANB-$NH_2$ or pNA, wherein ANB-$NH_2$ is 5-amino-2-benzoic acid amide, pNA is 4-nitroaniline, for use in the *in vitro* diagnosis of inflammation and/or neoplasm.

8.  Diagnostic marker defined by general formula $ABZ^1$-$Met^2$-$Lys^3$-$Val^4$-$Trp^5$-$X^6$ wherein ABZ denotes 2-aminobenzoic acid and X denotes ANB-$NH_2$ or pNA, where ANB-$NH_2$ is 5-amino-2-benzoic acid amide, pNA is 4-nitroaniline, for use in the *in vitro* diagnosis of neoplasm.

9.  Diagnostic kit for use in the *in vitro* diagnosis of epithelial neoplasm and/or inflammation comprising at least one compound defined by the general formula $ABZ^1$-$Leu^2$-$Glu^3$-$Pro^4$-$Val^5$-$X^6$ and at least one compound defined by the general formula $ABZ^1$-$Met^2$-$Lys^3$-$Val^4$-$Trp^5$-$X^6$, wherein: ABZ denotes 2-aminobenzoic acid, X denotes ANB-$NH_2$ or pNA, wherein ANB-$NH_2$ is 5-amino-2-benzoic acid amide, pNA is 4-nitroaniline.

10.  Method for the *in vitro* diagnosing of epithelial neoplasm and/or inflammatory process in a mammalian biological material, **characterized in that** at least one compound defined by the claim 1 is for use as the diagnostic marker, wherein at least one compound is added to the biological material and then the absorbance is measured at wavelengths in the range from 300 to 500 nm.

11.  The method according to the claim 10, wherein the absorbance measurements are made at 380-430 nm.

12.  Method for the *in vitro* diagnosing of epithelial neoplasm in mammalian biological material, **characterized in that** at least one compound defined by the claim 2 is for use as the diagnostic marker, wherein the compound is added to the biological material, and then the absorbance at a wavelength in the range of 300 to 500 nm is measured.

13.  The method according to the claim 12, wherein the absorbance measurements are made at 380-430 nm.

14.  Method for the *in vitro* diagnosing and differentiating between epithelial neoplasm and an inflammatory process in a mammalian biological material, **characterized in that**, at least one compound defined by the claim 1 and at least one compound defined by the claim 2 are for use as the diagnostic marker, wherein the compounds are added to the biological material and then the absorbance at a wavelength in the range of 300 to 500 nm is measured.

15.  The method according to the claim 14, **characterized in that** the absorbance measurements are made at 380-430 nm.

**Patentansprüche**

1.  Ein Molekül, das durch eine allgemeine Formel: $ABZ^1$-$Leu^2$-$Glu^3$-$Pro^4$-$Val^5$-$X^6$ definiert ist, wobei ABZ für 2-Aminobenzoesäure, X für ANB-NH2 oder pNA steht, wobei ANB -$NH_2$ für 5-Amino-2-Benzoesäure und pNA für 4-Nitroanilin steht.

2.  Ein Molekül, das durch eine allgemeine Formel: $ABZ^1$-$Met^2$-$Lys^3$-$Val^4$-$Trp^5$-$X^6$ definiert ist, wobei ABZ für 2-Aminobenzoesäure und X für ANB-$NH_2$ oder pNA steht, wobei ANB -$NH_2$ für 5-Amino-2-Benzoesäure und pNA für 4-Nitroanilin steht.

3.  Verfahren zur Synthese des Moleküls, das durch die Formel $ABZ^1$-$Leu^2$-$Glu^3$-$Pro^4$-$Val^5$-ANB-$NH_2^6$ definiert ist, wobei ABZ für 2-Aminobenzoesäure und ANB -$NH_2$ für 5-Amino-2-Benzoesäureamid steht, ist **dadurch gekenn-**

**zeichnet, dass** das Molekül durch Synthese auf festem Träger erhalten wird, die in den folgenden Schritten durchgeführt wird:

a) Vorbereitung des Harzes, das die Umwandlung von Säure zu Amid ermöglicht,

b) Einschätzung der Schutzgruppe,

c) Ablagerung von ANB 5-Amino-2-Nitrobenzoesäure,

d) Schritte von b) bis c) sind zu wiederholen, bis ein Molekül erhalten wird, in dem die Synthese vom Rest von 6 bis 1 durchgeführt wird und das resultierende Peptid dann vom Harz getrennt wird.

4. Verfahren zur Synthese des Moleküls, das durch die Formel $ABZ^1$-$Leu^2$-$Glu^3$-$Pro^4$-$Val^5$-ANB-$pNA^6$ definiert ist, wobei ABZ für 2-Aminobenzoesäure und pNA für 4-Nitroanilin steht, ist **dadurch gekennzeichnet, dass** das Molekül durch Synthese auf festem Träger erhalten wird, die in den folgenden Schritten durchgeführt wird:

a) Vorbereitung von 2-Chlorochlorotrityl-Harz,

b) Entschützung der Schutzgruppe,

c) Deposition von Pro4 auf dem Harz,

d) Schritte von b) bis c) sind zu wiederholen, bis ABZ-Leu-Glu-Pro erhalten wird,

e) Abtrennung des erhaltenen Peptids vom Harz,

f) Erhalt von para-Nitroanilid der Val-pNA Aminosäure,

g) Verbindung von para-Nitroanilid mit geschütztem Peptid ABZ-Leu-Glu-Pro + Val-pNa,

h) Entfernung der Seitengruppen von Aminosäuren.

5. Verfahren zur Synthese des Moleküls, das durch die Formel $ABZ^1$-$Met^2$-$Lys^3$-$Val^4$-$Trp^5$-ANB-$NH_2^6$ definiert ist, wobei ABZ für 2-Aminobenzoesäure und ANB-$NH_2$ für 5-Amino-2-Benzoesäureamid steht, ist **dadurch gekennzeichnet, dass** das Molekül durch Synthese auf festem Träger erhalten wird, die in den folgenden Schritten durchgeführt wird:

a) Vorbereitung des Harzes, das die Umwandlung von Säure zu Amid ermöglicht,

b) Entschützung der Schutzgruppe,

c) Deposition von ANB 5-Amino-2-Nitrobenzoesäure,

d) Schritte von b) bis c) sind zu wiederholen, bis ein Molekül erhalten wird, in dem die Synthese vom Rest von 6 bis 1 durchgeführt wird und das resultierende Peptid dann vom Harz getrennt wird.

6. Verfahren zur Synthese des Moleküls, das durch eine allgemeine Formel $ABZ^1$-$Met^2$-$Lys^3$-$Val^4$-$Trp^5$-$pNA^6$ definiert ist, wobei ABZ für 2-Aminobenzoesäure und pNA für 4-Nitroanilin steht, ist **dadurch gekennzeichnet, dass** das Molekül durch Synthese auf festem Träger erhalten wird, die in den folgenden Schritten durchgeführt wird:

a) Vorbereitung von 2-Chlorochlorotrityl-Harz,

b) Entschützung der Schutzgruppe,

c) Deposition von Pro4 auf dem Harz,

d) Schritte von b) bis c) sind zu wiederholen, bis $ABZ^1$-$Met^2$-$Lys^3$-$Val^4$ erhalten wird,

e) Abtrennung des erhaltenen Peptids vom Harz,

f) Erhalt von para-Nitroanilid der Trp-pNA Aminosäure,

g) Verbindung von para-Nitroanilid mit geschütztem Peptid (ABZ-Leu-Glu-Pro + Trp-pNa),

h) Entfernung der Seitengruppen von Aminosäuren.

7. Diagnosemarker, der durch eine allgemeine Formel $ABZ^1$-$Leu^2$-$Glu^3$-$Pro^4$-$Val^5$-$X^6$ definiert ist, wobei ABZ für 2-Aminobenzoesäure, X für ANB-$NH_2$ oder pNA steht, wobei ANB -$NH_2$ für 5-Amino-2-Benzoesäureamid und pNA für 4-Nitroanilin steht, zur Anwendung bei der in vitro Diagnostik von Entzündungen und/oder Krebs.

8. Diagnosemarker, der durch eine allgemeine Formel $ABZ^1$-$Met^2$-$Lys^3$-$Val^4$-$Trp^5$-$X^6$ definiert ist, wobei ABZ für 2-Aminobenzoesäure, X für ANB-$NH_2$ oder pNA steht, wobei ANB -$NH_2$ für 5-Amino-2-Benzoesäureamid und pNA für 4-Nitroanilin steht zur Anwendung bei der in vitro Diagnostik von Krebs.

9. Diagnosekit zur Anwendung bei der in vitro Diagnose von Epithelkrebs und/oder Entzündungen, enthaltend mindestens ein Molekül, das durch eine allgemeine Formel $ABZ^1$-$Leu^2$-$Glu^3$-$Pro^4$-$Val^5$-$X^6$ definiert ist und mindestens ein Molekül, das durch eine allgemeine Formel $ABZ^1$-$Met^2$-$Lys^3$-$Val^4$-$Trp^5$-$X^6$ definiert ist, wobei ABZ für 2-Aminobenzoesäure, X für ANB-$NH_2$ oder pNA steht, wobei ANB -$NH_2$ für 5-Amino-2-Benzoesäureamid und pNA für 4-Nitroanilin steht.

10. Verfahren zur Diagnose von in vitro Epithelkrebs und/oder Entzündungen in biologischem Säugetiermaterial dieses Verfahren ist **dadurch gekennzeichnet, dass** mindestens ein vom Anspruch 1 des definiertes Molekül als ein Diagnosemarker verwendet wird, bei dem mindestens ein Molekül dem biologischen Material zugesetzt wird und dann die Absorption für Wellenlängen zwischen 300 und 500 nm gemessen wird.

11. Verfahren nach Anspruch 10 bei dem Messungen für Wellenlängen zwischen 380 und 430 nm.

12. Verfahren zur Diagnose von in vitro Epithelkrebs in biologischem Säugetiermaterial. Dieses Verfahren ist **dadurch gekennzeichnet, dass** mindestens ein vom Anspruch definiertes Molekül als ein Diagnosemarker verwendet wird, bei dem mindestens ein Molekül dem biologischen Material zugesetzt wird und dann die Absorption für Wellenlängen zwischen 300 und 500 nm gemessen wird.

13. Verfahren nach Anspruch 12 bei dem Messungen für Wellenlängen zwischen 380 und 430 nm.

14. Verfahren zur Diagnose und in-vitro-Differenzierung zwischen Epithelkrebs und Entzündung in biologischem Säugetiermaterial. Dieses Verfahren ist **dadurch gekennzeichnet, dass** mindestens ein vom 1. Punkt des Patenanspruchs definiertes Molekül und mindestens nach Anspruch 2 definiertes Molekül als Diagnosemarker verwendet werden, bei dem Moleküle dem biologischen Material zugesetzt werden und dann die Absorption für Wellenlängen zwischen 300 und 500 nm gemessen wird.

15. Verfahren, nach Anspruch 14 bei dem Messungen für Wellenlängen zwischen 380 und 430 nm.

**Revendications**

1. Molécule définie par la formule générale suivante : $ABZ^1$-$Leu^2$-$Glu^3$-$Pro^4$-$Val^5$-$X^6$, où ABZ signifie acide 2-amino-benzoïque, X signifie $ANB$-$NH_2$ ou pNA, dans lequel $ANB$-$NH_2$ signifie acide 5-amino-2-benzoïque, et pNA 4-nitroaniline.

2. Molécule définie par la formule générale suivante: $ABZ^1$-$Met^2$-$Lys^3$-$Val^4$-$Trp^5$-$X^6$, où ABZ signifie acide 2-amino-benzoïque, X signifie $ANB$-$NH_2$ ou pNA, dans lequel $ANB$-$NH_2$ signifie acide 5-amino-2-benzoïque, et pNA 4-nitroaniline.

3. La méthode de synthèse de la molécule définie par la formule $ABZ^1$-$Leu^2$-$Glu^3$-$Pro^4$-$Val^5$-$ANB$-$NH_2^6$, où ABZ signifie l'acide 2-aminobenzoïque, $ANB$-$NH_2$ signifie amid de l'acide 5-amino-2-benzoïque, est caractéristique en ce que la molécule s'obtient à la suite d'une synthèse sur le support solide effectuée dans les étapes suivantes:

   a) préparation de la résine permettant la conversion de l'acide en l'amide,
   b) déprotection du groupe protecteur,
   c) fixation de l'acide 5-amino-2-benzoïque ANB,
   d) etapes répétées de b) à c) jusqu'à l'obtention de la molécule, où la synthèse est effectuée à parftir des restes de 6 à 1, et le peptide obtenu est ensuite séparé de la résine.

4. La méthode de synthèse de la molécule définie par la formule $ABZ^1$-$Leu^2$-$Glu^3$-$Pro^4$-$Val^5$-$ANB$-$pNA^6$, où ABZ signifie l'acide 2-aminobenzoïque, et pNA 4-nitroaniline, est caractéristique en ce que la molécule s'obtient à la suite d'une synthèse sur le support solide effectuée dans les étapes suivantes:

   a) préparation de la résine de 2-chlorochlorotrityle,
   b) déprotection du groupe protecteur,
   c) fixation de Pro4 sur la résine,
   d) étapes répétées de b) à c) jusqu'à l'obtention de l'ABZ-Leu-Glu-Pro;
   e) séparation du peptide obtenu de la résine,
   f) obtention du paranitroanilide de l'acide aminé Val-pNA
   g) liaison du paranitroanilide avec le peptide protégé ABZ-Leu-Glu-Pro + Val-pNa,
   h) élimination des groupes latéraux des acides aminés.

5. La méthode de synthèse de la molécule définie par la formule ABZ$^1$-Met$^2$-Lys$^3$-Val$^4$-Trp$^5$-ANB-NH$_2$$^6$, où ABZ signifie l'acide 2-aminobenzoïque, ANB-NH$_2$ signifie l'amide de l'acide 5-amino-2-benzoïque, est caractéristique en ce que la molécule s'obtient à la suite d'une synthèse sur le support solide effectuée dans les étapes suivantes:

    a) préparation de la résine permettant la conversion de l'acide en l'amide,
    b) déprotection du groupe protecteur,
    c) fixation de l'acide 5-amino-2-benzoïque ANB,
    d) étapes répétées de b) à c) jusqu'à l'obtention de la molécule, où la synthèse est effectuée à parftir des restes de 6 à 1, et le peptide obtenu est ensuite séparé de la résine.

6. La méthode de synthèse de la molécule définie par la formule générale ABZ$^1$-Met$^2$-Lys$^3$-Val$^4$-Trp$^5$-pNA$^6$, où ABZ signifie l'acide 2-aminobenzoïque, et pNA 4-nitroaniline, est caractéristique en ce que la molécule s'obtient à la suite d'une synthèse sur le support solide effectuée dans les étapes suivantes:

    a) préparation de la résine de 2-chlorochlorotrityle,
    b) déprotection du groupe protecteur,
    c) fixation de Val4 sur la résine,
    d) étapes répétées de b) à c) jusqu'à l'obtention du ABZ1-Met2-Lys3-Val,
    e) séparation du peptide obtenu de la résine,
    f) obtention du paranitroanilide de l'acide aminé Trp-pNA
    g) liaison du paranitroanilide avec le peptide protégé (ABZ-Leu-Glu-Pro + Trp-pNa),
    h) élimination des groupes latéraux des acides aminés.

7. Marqueur cible définie par la formule générale suivante: ABZ$^1$-Leu$^2$-Glu$^3$-Pro$^4$-Val$^5$-X$^6$, où ABZ signifie l'acide 2-aminobenzoïque, X signifie ANB-NH$_2$ ou pNA, dans lequel ANB-NH$_2$ signifie l'amide de l'acide 5-amino-2-benzoïque, et pNA 4-nitroaniline, pour utilisation dansla dans le diagnostic *in vitro* des états inflammatoires et/ou des cancers.

8. Marqueur cible définie par la formule générale suivante: ABZ$^1$-Met$^2$-Lys$^3$-Val$^4$-Trp$^5$-X$^6$, où ABZ signifie l'acide 2-aminobenzoïque, X signifie ANB-NH$_2$ ou pNA, dans lequel ANB-NH$_2$ signifie l'amide de l'acide 5-amino-2-benzoïque, et pNA 4-nitroaniline, pour utilisation dans le diagnostic des cancers *in vitro*.

9. Kit de diagnostic à utiliser dans le diagnostic des cancers des épithéliums *in vitro* et/ou des états inflammatoires contenant au moins une particule définie par la formule générale ABZ$^1$-Leu$^2$-Glu$^3$-Pro$^4$-Val$^5$-X$^6$ et moins une particule définie par la formule générale ABZ$^1$-Met$^2$-Lys$^3$-Val$^4$-Trp$^5$-, où ABZ signifie l'acide 2-aminobenzoïque, X signifie ANB-NH2 ou pNA, dans lequel ANB-NH$_2$ signifie l'amide de l'acide 5-amino-2-benzoïque, et pNA 4-nitroaniline.

10. Méthode de diagnostic *in vitro* des cancers des épithéliums et/ou des états inflammatoires dans la matière biologique mammifère , caractéristique en ce que, au moins une particule définie dans la revendication 1 est utilisée comme marqueur cible, dans lequel au moins une particule est ajoutée à la matière biologique et ensuite l'absorbance est mesurée pour l'onde dont la longueur s'inscrit dans la plage de 300 à 500 nm.

11. Méthode selon la revendication 10 est **caractérisée en ce qu'**il est, les mesures sont effectuées dans la plage de 380 à 430 nm.

12. Méthode de diagnostic des cancers des épithéliums *in vitro* dans la matière biologique mammifère , caractéristique en ce que, au moins une particule définie dans la revendication 2 est utilisée comme marqueur cible, dans lequel au moins une particule est ajoutée à la matière biologique et ensuite l'absorbance est mesurée pour l'onde dont la longueur s'inscrit dans la plage de 300 à 500 nm.

13. Méthode selon la revendication 12 est **caractérisée en ce qu'**il est les mesures sont effectuées dans la plage de 380 à 430 nm.

14. Méthode de diagnostic et de différenciation *in vitro* entre le cancers de l'épithéliums et le processus inflammatoire dans la matière biologique mammifère, caractéristique en ce que, au moins une particule définie dans la revendication 1 et au moins une particule définie dans la revendication 2, sont utilisées comme marqueurs cibles, où les particules sont ajoutées à la matière biologique et ensuite l'absorbance est mesurée pour l'onde dont la longueur s'inscrit dans la plage de 300 à 500 nm.

**15.** Méthode selon la revendication 14 est **caractérisée en ce que** les mesures sont effectuées dans la plage de 380 à 430 nm.

Fig.1.

Fig.2.

Fig.3.

Fig. 4

Fig. 5.

Fig.6

Fig. 7

Fig.8.

Fig.9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011038142 A

- WO 2014042209 A

**Non-patent literature cited in the description**

- **GRUBA et al.** *FEBS Journal,* 2016, vol. 283, 2929-2948